(19) Europäisches Patentamt
European Patent Office
Office européen des brevets

(11) Publication number: **0 281 316 A2**

(12) **EUROPEAN PATENT APPLICATION**

(21) Application number: **88301609.9**

(51) Int. Cl.⁴: **C07K 5/00**, A61K 37/64

(22) Date of filing: **25.02.88**

(30) Priority: **27.02.87 JP 46454/87**
**12.05.87 JP 115144/87**
**18.08.87 JP 206146/87**
**16.11.87 JP 289017/87**

(43) Date of publication of application:
**07.09.88 Bulletin 88/36**

(84) Designated Contracting States:
**AT BE CH DE ES FR GB GR IT LI LU NL SE**

(71) Applicant: **YAMANOUCHI PHARMACEUTICAL CO. LTD.**
**No. 3-11 Nihonbashi-Honcho, 2-chome**
**Chuo-ku**
**Tokyo(JP)**

(72) Inventor: **Nakano, Kohji**
**1086-1, Sanagaya Shiraoka-cho**
**Minamisaitama-gun Saitama(JP)**
Inventor: **Fujikura, Takashi**
**3-17, Shirhata 4-chome**
**Urawa-shi Saitama(JP)**
Inventor: **Hara, Ryuichiro**
**3-19-11,Honcho Nakano-ku**
**Tokyo(JP)**
Inventor: **Ichihara, Masato**
**3-16-1, Hasune Itabashi-ku**
**Tokyo(JP)**
Inventor: **Fukunaga, Yikiko**
**3-19-6, Nakajujo Kita-ku**
**Tokyo(JP)**
Inventor: **Shibasaki, Masayuki**
**3-27-B1107, Nakadai Itabashi-ku**
**Tokyo(JP)**

(74) Representative: **Geering, Keith Edwin et al**
**REDDIE & GROSE 16 Theobalds Road**
**London WC1X 8PL(GB)**

(54) **Renin inhibitors.**

(57) Novel Compounds of the following formula (I) and their salts

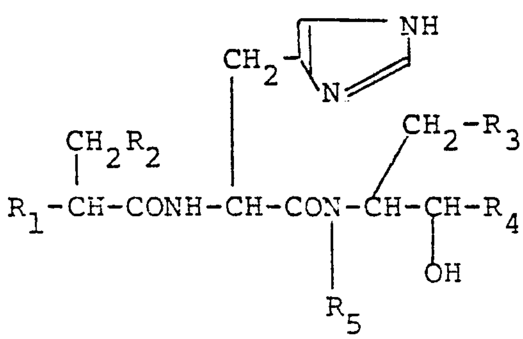

(I)

EP 0 281 316 A2

are useful as renin inhibitors and hence as medical agents - in particular, anti-hypertensive agents.

# RENIN INHIBITORS

This invention relates to renin inhibitors. Renin is an aspartic proteinase which catalyzes specific hydrolysis of the glycoprotein angiotensinogen to give the decapeptide antiogensin I. A. dipeptidylcarboxypeptidase, antiotensin converting enzyme (ACE), then converts it to the octapeptide AII, which, in addition to being an extremely potent vasoconstrictor, is also a promotor of aldosterone release and thereby sodium retention. Aminopeptidases further act on AII to give AIII, which produces effects similar to those produced by AII, but to a lesser extent. This cascade, known as the renin-angiotensin system, is therefore important in the regulation of blood pressure and electrolyte homeostasis. Renin inhibitors inhibit reaction between renin and angiotensinogen, and reduce the formation of angiotensin I and so inhibit the formation of angiotensin II (AII) which produces high blood pressure and is a promotor of aldosterone release. Since renin has remakable speficity toward its subtrate, inhibitors of this enzyme should produce a more direct probe of the renin-angiotensin system. In a limited number of cases, the manipulation of the renin-antiogtension system by renin inhibitors (among them substrate analogue inhibitor, peptides derived from the profragment segment, pepstatin/statin-based inhibitors, and other transition-state or intermediate substrate analogues has led to blood-pressure lowering. Such recently found renin inhibitors are shown in J. Med. Chem., 28, 1553-(1985), J, Med, Chem., 28, 1756(1985), and Japanese Patent Specifications Nos.61-33152, 61-122296. 61-236770, 61-275257 and 61-275258.

Renin inhibitors suitable for clinical use should have :

(1) strong inhibiting effect on human renin;

(2) prolonged activity;

(3) good absorption through the intestine; and

(4) high specific activity in inhibiting human renin. Some transition-state substrate analogue (statin-based) inhibitors have some inhibiting effect, and some peptides having N-terminal and/or C-terminal protecting groups have shown prolongation of the effect to some extent; however, the above requirements (1) to (4) have not been met simultaneously in the prior art. For improved absorption through the intestine, low molecular compounds have been thought to be necessary, and research has been proceeding for providing low molecular tripeptide and dipeptide transition state analogues; however, prior low molecular weight transition-state analogue renin inhibitors suffer considerable loss of activity by the action of proteinase in the alimentary canal system. Only the above Japanese 61-236770 describes renin inhibitor peptide being absorbed to some extent after oral administration, but this inhibitor, has poor specific activity for human renin and insufficiently prolonged action.

On the other hand, Japanese 61-33152 describes some compounds of the formula:

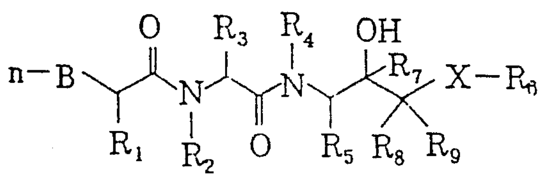

$R_6$ includes "heteroaryl" as a kind of aryl, but there is no specific disclosure of a hetero ring.

EP 172347 and 200406 disclose renin inhibitors such as

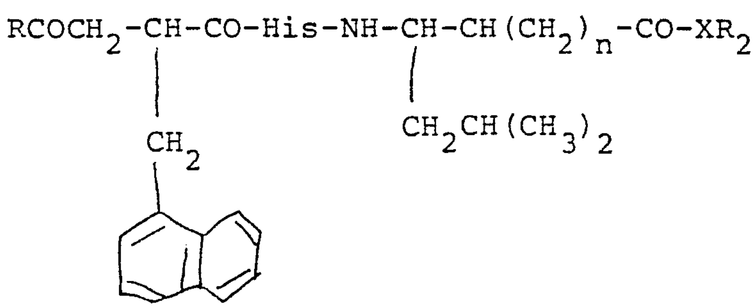

However, the compounds of the present invention (of formula (I) below ) are entirely different structurally

from these prior art compounds.

This invention provides compounds of formula (I), and salts thereof, which are useful as medical agents, e.g. anti-hypertensive agents.

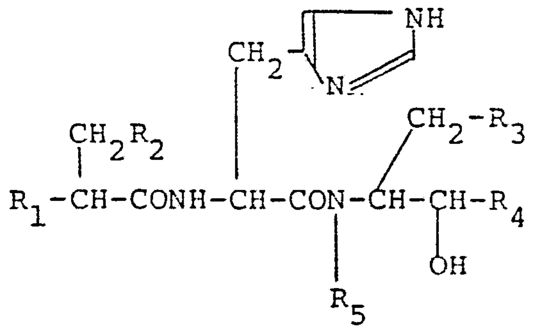

(I)

wherein $R_1$ is a (lower alkoxy)carbonyl group, a (lower alkoxy)carbonylamino group, a group of formula $-(CH_2)_n$-A-$R^a$ [wherein n is an integer of 1 or 2; A is a single bond between $-(CH_2)_n$ and $R^a$ (in this case $R_1$ is $-(CH_2)_n-R^a$), an alkylene group which may be substituted by hydroxy group(s), or carbonyl; and $R^a$ is a cyano group, a lower alkyl group, a lower alkynyl group, a cycloalkyl group, a cylcoalkylidene group, an aryl group, an aryloxy group which may be substituted by halogen atom(s), or a heterocyclic group which may be substituted by amino group(s)], or a group of formula -CONH-B-$R^b$ [wherein B is a single bond, or alkylene, and $R^b$ is hydroxyl, an aryl group, or a heterocyclic group]; $R_2$ is a phenyl group or a naphthyl group; $R_3$ is a lower alkyl group, a cyclohexyl group, or a phenyl group; $R_4$ is a nitromethyl group, a group of the formula -COO$R^c$ (wherein $R^c$ is a lower alkyl group), or a group of formula

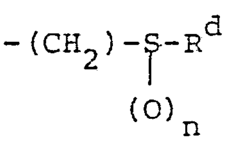

(wherein n is zero or an integer of 1 or 2; and $R^d$ is heterocyclic group which may be substituted by at least one group selected from lower alkyl, carbamoyl-lower-alkyl and hydroxy-lower-alkyl group]; and $R^5$ is a hydrogen atom or a lower alkyl group.

In this specification, the term "lower" refers to a straight or branched carbon chain having up to 6 carbon atoms, unless otherwise indicated. Accordingly, concrete examples of the lower alkyl group include methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, and 1-ethyl-2-methylpropyl groups, etc. A carbamoyl lower alkyl group is one obtainable by sustituting any hydrogen atom in a "lower alkyl" with "carbamoyl" ($-CONH_2$) and concrete examples include carbamoylmethy, 2-or 1-carbamoylethyl, 3-carbamoylpropyl, 2-carbamoylpropyl, 1-carbamoylpropyl, carbamoylpentyl, carbamoylhexyl, carbamoylbutyl, and their branched lower alkyl derivatives. "Cycloalkyl" means $C_3$ to $C_7$ ring alklyl, and concrete examples are cyclopropyl, cyclobutyl, cyclopentyl, cyclohexyl, cycloheptyl, etc. Examples of "lower alkynyl" are ethynyl, propynyl, etc.

Further as the "lower alkoxy group", mention may be made of methoxy, ethoxy, propoxy, isopropoxy, butoxy, isobutoxy, sec-butoxy, tert-butoxy, pentyloxy (amyloxy), isopentyloxy, tert-pentyloxy, neopentyloxy, 2-methylbutoxy, 1,2-dimethylpropoxy, 1-ethylpropoxy and hexyloxy groups and the like.

Concrete examples of the "aryloxy group" include phenoxy and naphthoxy groups and the like, with particular preference for phenoxy.

As an "aryl group", there are as concrete examples phenyl and naphthyl groups and the like but particularly preferred is phenyl.

An alkylene group preferably has 1 to 6 carbon atoms. Specific examples are methylene, ethylene,

methylmethylene

$$\overset{CH_3}{\overset{|}{(-CH-)}}\ ,$$

trimethylene, tetramethylene, 3,3-dimethyltetramethylene, 4,4-dimethyltetramethylene, 1,1,3-trimethyltrimethylene, 1,1,2-trimethyltrimethylene, 1,1,2,2-tetramethylethylene, 1,1-dimethyl-2-ethylethylene, 1,1-diethylethylene.

"Cycloalkylidene" is a $C_3$ to $C_7$ aliphatic divalent group, and concrete exmples are cyclopropylidene, cyclobutylidene, cyclopentylidene, cyclohexylidene, cycloheptylidene, etc.

"Heterocyclic group" means a monocyclic 5-or 6-membered heterocyclic ring. Concrete examples include such rings which contain oxygen atom(s) and may additionally contain other hetero atom(s) such as tetrahydrofuryl, dihydrofuryl, furyl, dioxolanyl, tetrahydropyranyl, dihydropyranyl, pyranyl, dioxanyl, dioxynyl (containing one or two oxygens), isoxazolidinyl, isooxazolynyl, isooxazolyl, oxazolydinyl, oxazolyl, morpholino, 4H-1,4-oxazinyl, 4H-1,3-oxazinyl, 2H-1,3-oxazinyl, 6H-1,2-oxazinyl, 4H-1,2-oxazinyl, 2H-1,2-oxazinyl (containing one oxygen and one nitrogen atom); further examples are monocyclic 5-and 6-membered heterocyclic rings which contain nitrogen atom(s) and may additionally contain other hetero atom(s) such as oxygen, sulfur atom(s), including piperazinyl, pyrrolyl, pyrrolinyl, pyrrolidinyl, pyridyl, piperidinyl,piperidyl. Examples of other heterocyclic ring groups are thienyl, thiopyranyl, etc. (S-containing heterocyclics), and thiazolyl, iso-thiazoly, thiazolinyl, etc. (S-and N-containing heterocyclics). Concrete examples of heterocyclic in the case of $R^d$ are, in particular, $C_5$ and $C_6$ N-containing heterocyclics which may also contain O-and/or S-atom(s) such as pyrrolyl, pyrrolinyl, pyrrolidinyl, pyridyl, dihydropyridyl, piperidyl, pyrazolyl, pyrazolinyl, pyrazolidinyl, imidazolyl, imidazolynyl, imidazodinyl, pyrazinyl, piperazinyl, pyrimidinyl, pyridazinyl, triazinyl tetrazolyl, etc. ($N_1$-to $N_4$-containing heterocyclics); oxazolyl, oxazolidinyl, isooxazolyl, isooxazolinyl, isooxazolidinyl, etc. (heterocyclics containing one oxygen atom and one nitrogen atom); thiazolyl, thiazolinyl, thiazolidinyl, isothiazolyl, isothiazolinyl, isothiazolidinyl, 1,3,4-thiadiazolyl, 1,2,4-thiadiazolyl, 1,2,5-thiadiazolyl, etc. ($N_1$-or $N_2$-and S-containing heterocyclics) Further examples of 5-and 6-membered heterocyclic rings are tetrahydrofuryl, dihydrofuryl, furyl, dioxolanyl, dioxolyl, tetrahydropyranyl, dihydropyranyl, pyranyl, dioxanyl, dioxynyl (containing one or two oxygens); thienyl, thiopyranyl (containing sulfur); thiazolyl, iso-thiazolyl, thiazolinyl, etc. (containing sulfur and nitrogen).

The present invention includes the salts of compounds (I), and examples of such salts include addition salts with inorganic acids such as mineral acids (e.g. hydrochloric, hydrobromic, hydroiodic, sulfuric, nitric and phosphoric acids and the like) and with various organic acids such as formic, acetic, oxalic, citric, succinic, fumaric, maleic, malic, tartaric, methanesulfonic and ethanesulfonic acids and the like. Acidic amino acids such as aspartic acid, glutamic acid also form salts according to the invention. As salts of the present invention, there are also salts with alkali (sodium, potassium, etc.), and alkaline earth metals (magnesium, calcium, etc.), and with organic bases e.g. diethylamine, triethylamine, diethanolamine, cyclohexylamine.

Some compounds according to the invention can evidently have various isomers such as optical isomers, diastereoisomers and geometrical isomers. The present invention includes isolated isomers and any mixtures thereof. In particular, the formula (I) compounds have 3 to 4 assymetric carbon atoms, and have optical isomers.

The formula (I) compounds of this invention can be prepared for example, by the following processes.

Process 1

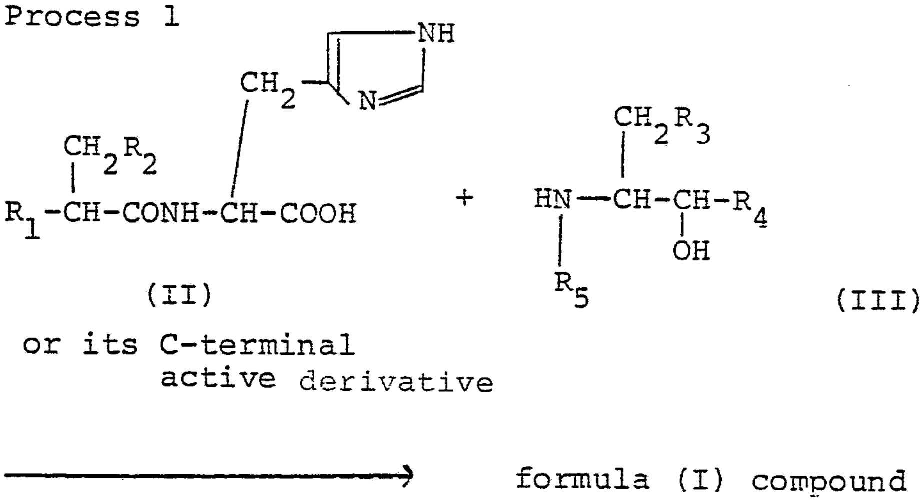

(II)

or its C-terminal active derivative

+

(III)

formula (I) compound

Thus formula (I) compound(s) can be prepared by reacting formula (II) compound(s) with formula (III) compound(s) and, if necessary, removing any protective group.

The formula (II) compounds may be used as the free acid or its active derivative. Coupling in the presence of condensing agent can be applied. Active esters such as phenol-active ester (N-nitrophenol-active ester, etc.), N-hydroxylamine-active ester (N-hydroxysuccinimide-active ester, 1-hydroxybenzotriazole-active ester, etc.), carbonic acid mono-alkyl ester, mixed-acid (organic acid) anhydride, mixed-phosphoric acid-active ester (diphenyl phosphoryl chloride and N-methylmorpholine can be used), acid azide (e.g prepared by acid hydrazide and nitrous acid), acid halides (acid chloride, acid bromide, etc.), and acid anhydride can be used as C-terminal active compound.

Coupling agents which can be used include N,N,'-dicyclohexylcarbodiimide, carbonyldiimidazole, diphenylphosphoryl azide (DPPA), etc. The reaction can usually be carried out in the presence of solvent, under cooling or at room temperature. As the solvent, there may be used organic solvent which does not take part in the reaction (for example, one or more of dimethylformamide, dimethylacetoamide, dioxane, tetrahydrofuran, ether, dichloromethane, dichloroethane, chloroform, carbon tetrachloride, dimethoxymethane, dimethoxyethane, ethyl acetate, benzene, acetonitrile, and dimethylsulfoxide).

Some active esters may (and some must) be reacted under anhydrous or dry conditions. It may be preferable to use N-methylmorpholine, triethylamine, trimethylamine, for a smooth reaction. That is, the presence of such base may result in a smooth reaction. Azide method or coupling method using diethylphosphorylcyanide, etc. are most preferable, in view of problems of protective groups and racemization. Protective groups are removed after the reaction, if they are used.

The preferred protective groups for carboxyl are the ester residues commonly used in the field of peptide synthesis - benzyl and substituted benzyls (p-nitrobenzyl, diphenylmethyl (benzohydryl), trityl, etc); lower alkyls (tert-butyl, methyl, ethyl, etc); phenacyl, 4-picolyl; cyclohexyl, etc. As a protective group for imidazolyl, there may be used benzyl, substitued benzyl as above, tert-butyl, etc.

Removal of the protective groups can be conducted by usual methods.

Process 2

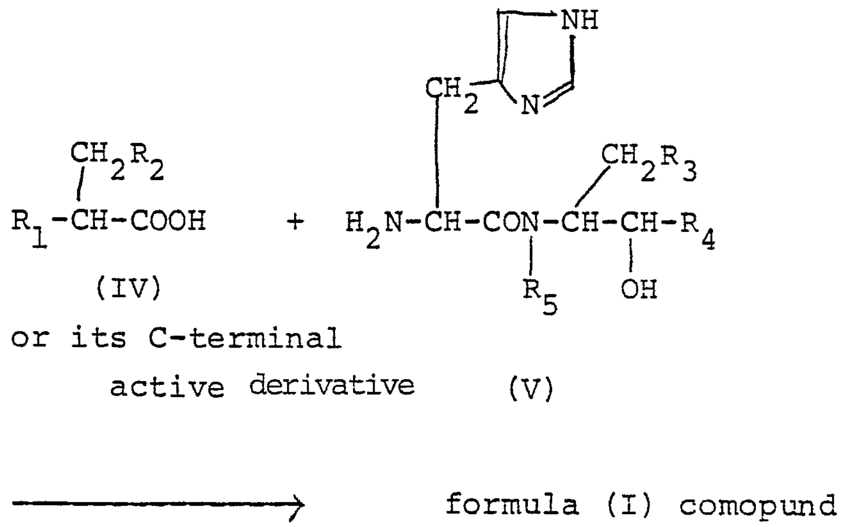

(IV)

or its C-terminal active derivative

(V)

formula (I) comopund

The formula (I) compounds of the present invention can thus also be prepared by reacting formula (IV) compound(s) and formula (IV) compound(s) and, if necessary, removing any protective group.

The reaction conditions, etc. are the same as in Process 1.

Other processes 3

According to the kinds of substituent in the formula (I) compound, there may be used various methods. For example, ester derivatives can be made by reacting the carboxylic acid or its reactive derivative with lower alcohol or lower alkyl halide (namely, alcohol-reactive ester) -usual esterification; and free carboxylic acid can be prepared by normal hydrolysis of ester derivatives.

When $R_1$ contains >N-CO-, the corresponding carboxylic acid or its C-terminal active ester may be used as a starting material and reacted with an amide derivative based upon the $R_1$ group.

The formula (I) compounds having a lower alkanoyl portion may be prepared by using the corresponding hydroxy derivative or its reactive derivative as a starting material, which is reacted with lower alkanecarboxylic acid or its reactive derivative.

In the case of $R_4$ being $-CH_2SR^d$, the following reaction method may be used

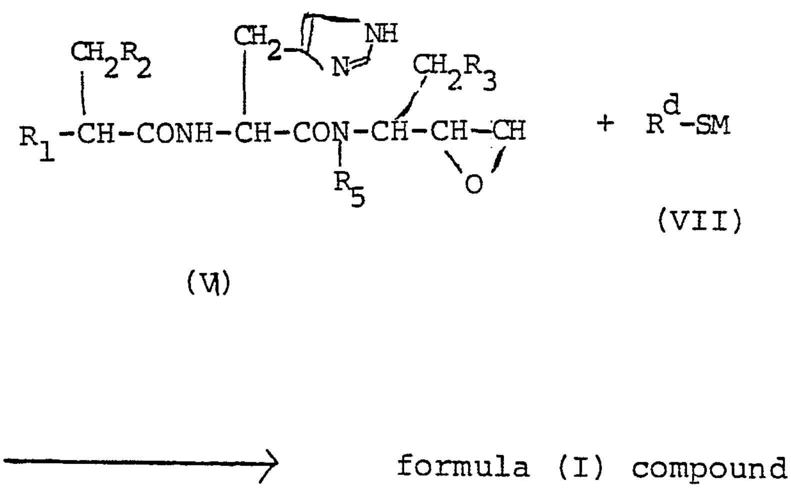

(VII)

(VI)

formula (I) compound

Thus, formula (I) compound(s) may be prepared by reacting formula (VI) compound(s) and formula (VII) compound(s). In this reaction scheme M represents hydrogen or an alkali metal atom - e.g. sodium, potassium, etc.

This reaction is preferably carried out in a solvent which does not take part in the reaction, and

examples of such solvent are N,N-dimethylformamide, dimethylsulfoxide, ether, tetrahydrofuran, dioxane, methanol, ethanol, iso-propanol, acetone, methylethylketone, dichloromethane, ethylene chloride, chloroform, carbon tetrachloride, etc. (organic solvents), water, and a mixture of water and organic solvent. When using a mercaptan compound as the formula (VI) compound, it is preferred to carry out the reaction in the presence of a base, and examples of the base are triethylamine, trimethylamine, potassium carbonate, sodium carbonate, potassium hydroxide, etc. (organic bases and inorganic bases). The reaction temperature is not particularly critical, and according to the starting materials, etc., the reaction can be conducted under cooling, at room temperature, or under heating. When $R_1$ contains $-COCH_2-$, the present compounds can be prepared from the corresponding carboxylic acid or its derivative and the corresponding halide or sulfonate ($X-CH_2-$where X=halogen or organic sulfonic acid residue e.g. p-toluenesulfonyloxy, methansulfonyl etc) in conventional manner.

For compounds (1) having a thioether portion, a usual thioether-formation reaction (e.g. between the corresponding mercaptan (---SH) or its alkali metal derivative and the corresponding halide or sulfonate) can be used.

The compounds of the present invention can be used as they are, in a free state, or as salts thereof. Isolation and purification can be performed by conventional chemical operations such as extraction, crystallization, recrystallization, various chromatography techniques, etc.

In the compounds of the present invention, racemates, optically active forms, diastereoisomers and the like may be present singly or in admixture. Stereochemically pure isomers can be obtained using appropriate raw compounds or by conventional racemic resolution [for example, by treatment with conventional optically active acid (tartaric acid, etc.) followed by optical resolution, etc.]. A mixture of diastereoisomers can be separated in conventional manner, for example, fractional crystallization, chromatography, etc.

The formula (I) compounds and their salts have strong inhibiting-activity specific to renin, prolonged action suitable for clinical administration, and good absorption through the intestine. Accordingly, the compounds of this invention are useful for treating and preventing high blood pressure, in particular, renin-angiotensin based high blood pressure. The superior effects of present compounds are shown by the following experiment.

(1) Inhibiting effect on human plasma renin.

To human plasma (250µl) having 0.5ng/ml/hr (37 °C)-angiotensin I forming activity, was added 225µl of an enzyme-inhibitor (BAL, 8-hydroxysulfate, pH4.6) and 25µl of a solution of the test compound in dimethylsulfoxide, and the mixture was stirred. A portion of the stirred mixture was subjected to incubation for 37 °C for 2 hours, and the rest of the mixture was kept at 4 °C. Taking 100µl of each of the mixtures, by measuring (by radio-immunoassay) the difference in the amounts of angiotensin I formed at 37 °C and 4 °C, the 50% inhibiting concentration IC50 (M) was calculated. Some date obtained are shown below.

| Ex. No. | $R_1$ | $R_2$ | $R_3$ | $R_4$ | $IC_{50}$/human plasma |
|---|---|---|---|---|---|
| Ex. 40 | 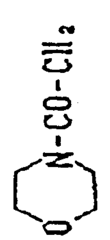 | 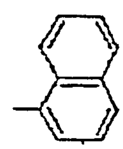 | 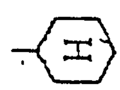 | 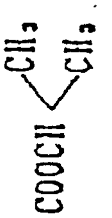 | $4 \times 10^{-9}$ |
| Ex. 7 | 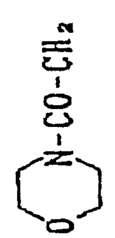 | 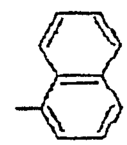 | 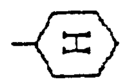 | $CH_2$-S-TZ | $5 \times 10^{-10}$ |
| Ex. 9 | $(CH_3)_2COCONH$- | 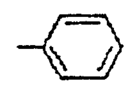 | 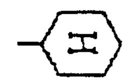 | $CH_2$-S-TZ | $4 \times 10^{-9}$ |

Tz: 1-methyl-5-tetrazolyl

Compositions containing one or more compounds (I) or salts thereof as effective components can be prepared as tablets, powders, granules, granulates, capsules, pills, liquid, injections, suppositories, ointments, etc. using conventional carriers, excipients, etc., and other additives, and can be administered orally (including sublingually) or parenterally.

The carriers and excipients for medical preparations include solid or liquid non-toxic pharmaceutical substances. Examples of such substances are lactose, magnesium stearate, starch, talc, gelatin, agar, pectin, gum arabic, olive oil, sesame oil, cacao butter, ethylene glycol, etc. and other substances ordinarily used.

Clinical dosage of the compounds of the present invenion may be appropriately determined depending

upon condition, body weight, age, sex, etc. of the patient to be treated, but is generally 0.5 to 5000 mg daily for an adult, administered in one dose or two sub-doses (oral:1 - 5000mg, parenteral: 0.5-1000mg).

The present compounds having peptide character can usually be administered as a pharmaceutical composition such as tablets, pills, capsules, etc. prepared using carriers or excipients usual for peptide-pharmaceutical compositions for oral or parenteral administration such as injection.

Some intermediate compounds used for preparation of compounds of the invention are novel. In the following Reference Examples various intermediates, including novel compounds, of use in the invention are prepared in conventional manner.

## REFERENCE EXAMPLES

### Reference Example 1

1. (2RS,3S)-3-N-t-butoxycarbonylamino-5-methyl-1-(1-methyl-5-tetrazolylthio)-2-hexanol.

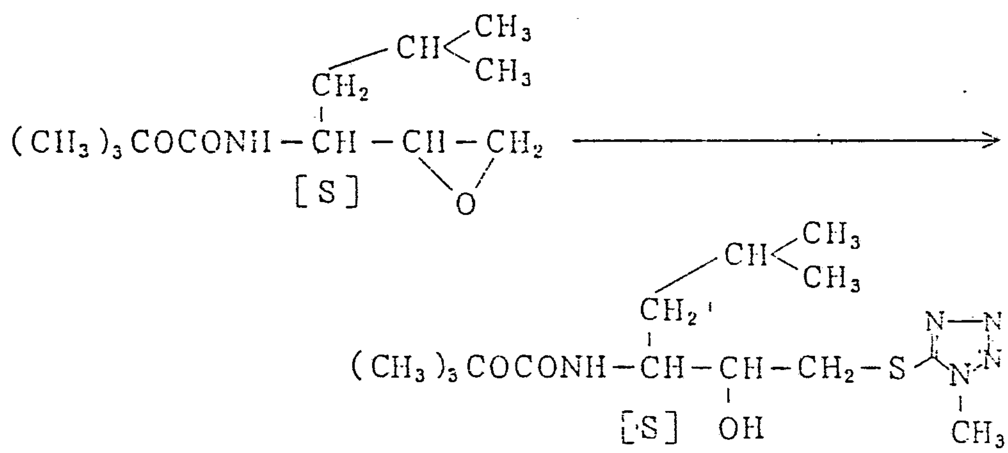

(m/z) : 346 ($M^+ + 1$), 290,246

(KBr) cm-1 : 3368,1688,

($CDCl_3$, TMS

δ ppm : 0.94 (d, 6H, 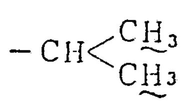 )

1.42 (s, 9H, t-Bu O- )

3.40 (d, 2H, 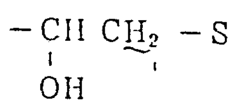 )

3.96 (s, 3H, 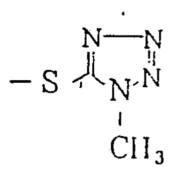 )

Reference Examples 2 - 5

In similar manner to 1 above, the following were prepared:

2. (2RS-3S)-3-N-t-butoxycarbonylamino-5-methyl-1-(2-thiazolythio)-2-hexanol.

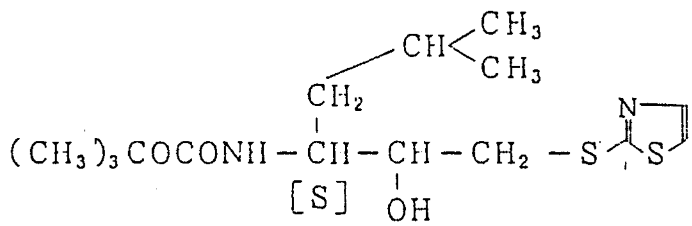

3. (2RS,3S)-3-N-t-butoxycarbonylamino-5-methyl-1-[5-methyl-1,3,4-thiadiazole-2-yl)thio]-2-hexanol.

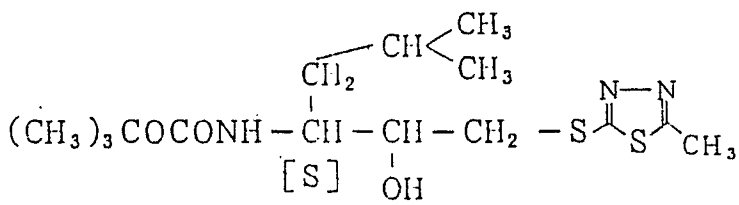

4. (2RS,3S)-3-N-t-butoxycarbonylamino-5-methyl-1-(4-methyl-2-oxazolythio)-2-hexanol.

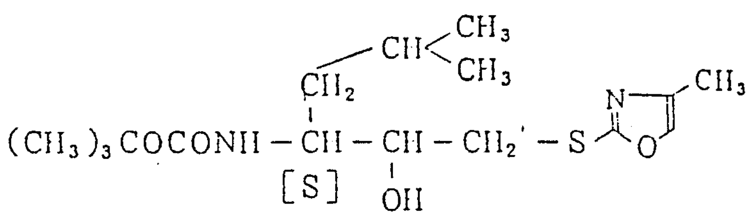

5. (2RS,3S)-3-N-t-butoxycarbonylamino-5-methyl-1-(1-methyl-2-imidazolythio)-2-hexanol.

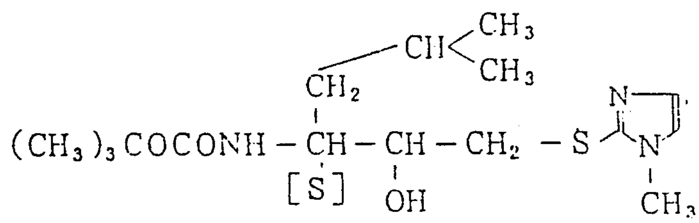

Reference Example 6

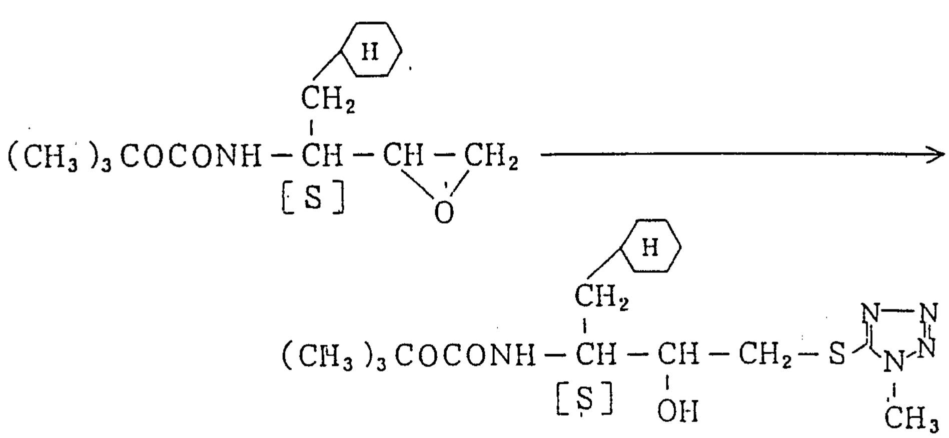

Reference Example 7

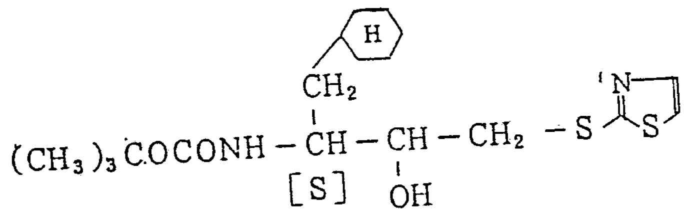

Reference Example 8

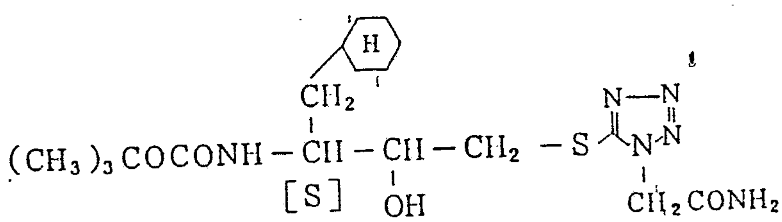

Reference Example 9

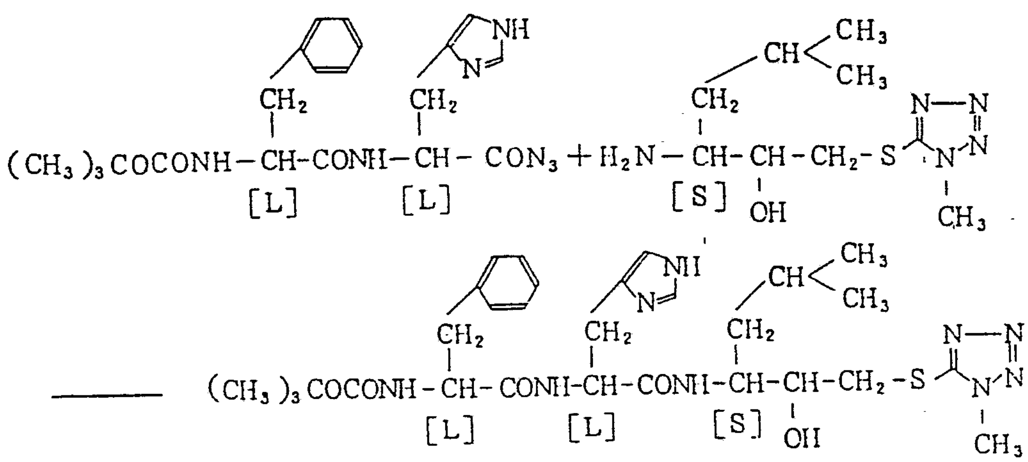

Reference Examples 10 to 16

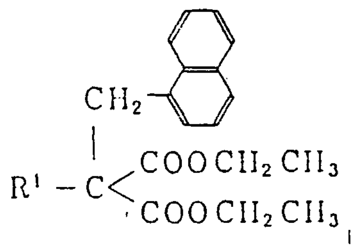

10. 2-(1-naphthylmethyl)-2-(phenylcarbonyl-methyl)-malonic acid diethyl ester.

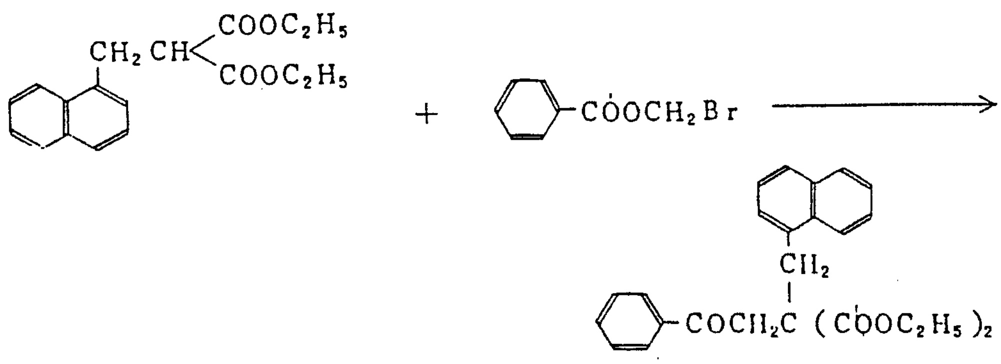

11. 2-(1-naphthylmethyl)-2-[2-oxo-2-(tetrahydropyran-4-yl)ethyl]malonic acid diethyl ester.
12. 2-[2-(3-thienyl)-2-oxoethyl]-2-(1-naphthylmethylmalonic acid diethyl ester.
13. 2-[2-(2-thienyl)-2-oxethyl]-2-(1-naphthylmethyl)malonic acid diethyl ester.
14. 2-(3-methyl-2-oxobutyl)-2-(naphthylmethyl) malonic acid diethyl ester.
15. 2-(3-propargyl)-2-(1-naphthylmethyl)malonic acid.
16. 2-[-2-(1,3-dioxolan-2-yl)ethyl]-2-(1-naphthylmethyl)malonic acid diethyl ester.

## Reference Examples 17 to 24

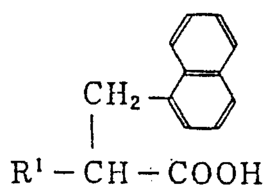

17. 2-(1-naphthylmethyl)-4-phenyl-4-oxobutyric acid
18. 4-cyclohexyl-2-(1-naphthylmethyl)-4-oxobutyric acid
19. 2-(1-naphthylmethyl)-4-oxo-4-(tetrahydropyran-4-yl)-butyric acid
20. 4-(3-thienyl)-2-(1-naphthylmethyl)-4-oxobutyric acid
21. 4-(2-thienyl)-2-(1-naphthylmethyl)-4-oxobutyric acid
22. 5-methyl-2-(1-naphthylmethyl)-4-oxohexanoic acid
23. 2-(1-naphthylmethyl)-4-pentinic acid
24. 4-(1,3-dioxolan-2-yl)-2-(1-naphthylmethyl)butyric acid

Reference Examples 25 to 32

25. 2-(1-naphthylmethyl)malonic acid monoethyl ester

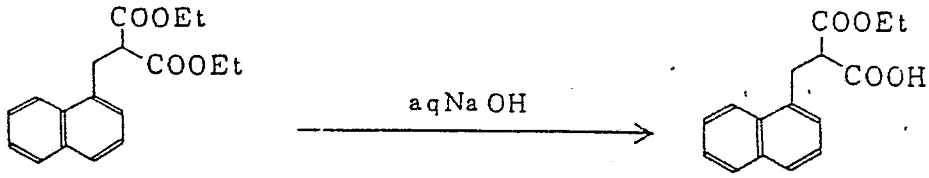


26.

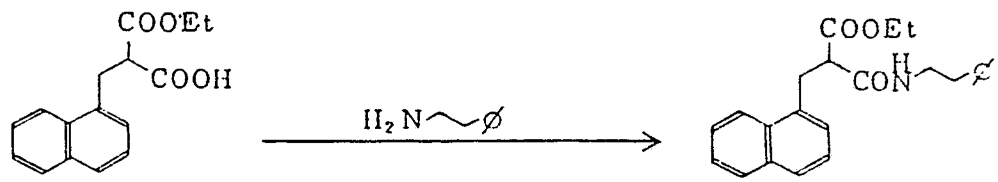

Reference Example 27

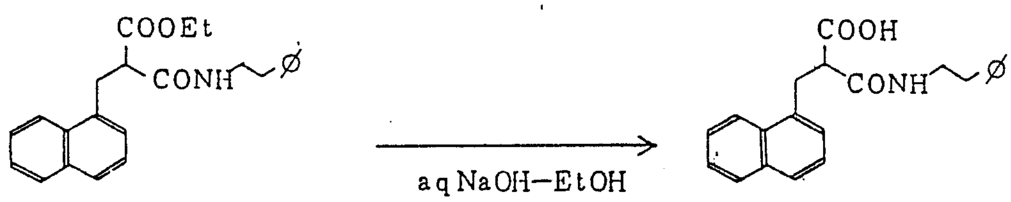


Reference Example 28

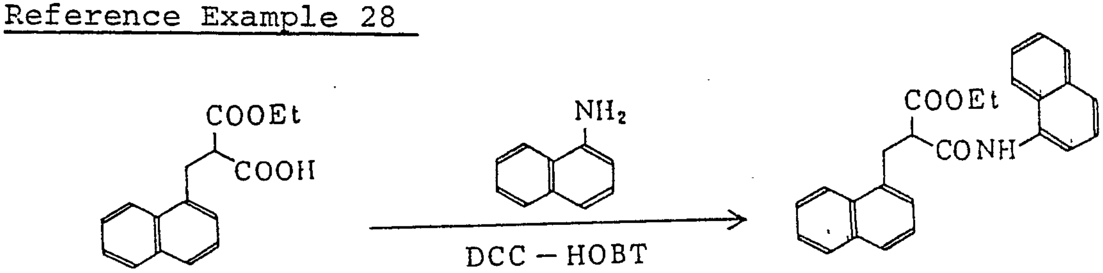


Reference Example 29

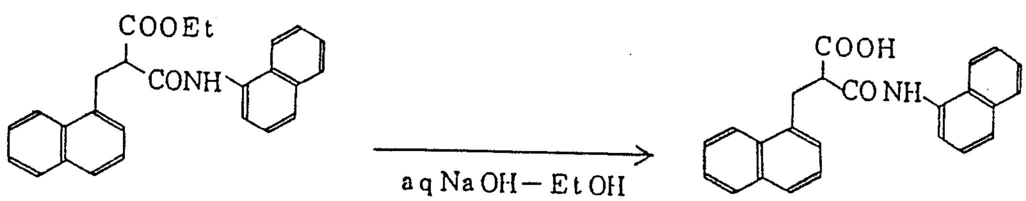

Reference Example 30

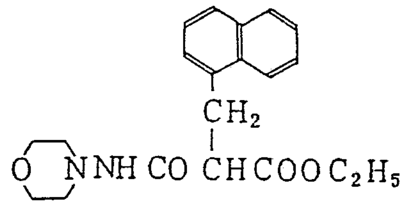

Reference Example 31

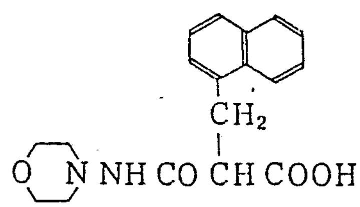

Reference Examples 32(a) and 32(b)

. 32(a)

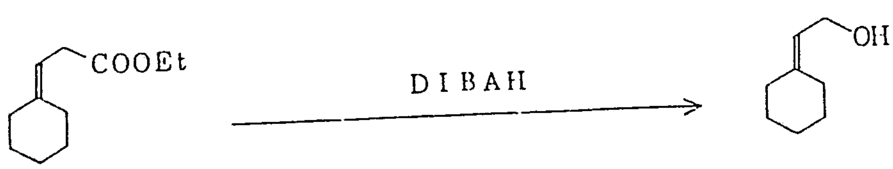

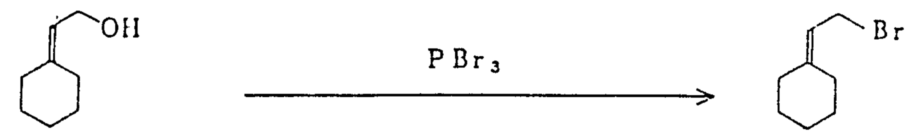


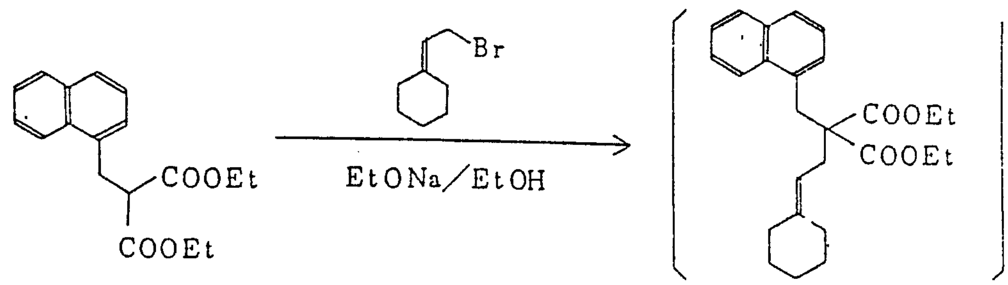


32(b)

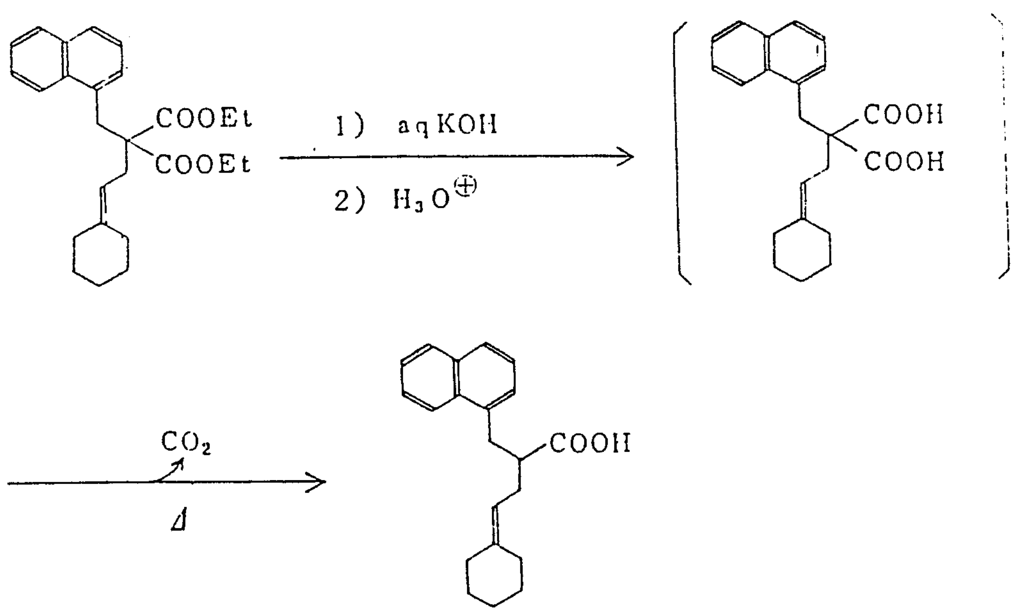

Reference Example 33

(2RS,3S)-3-amino-5,5-dimethyl-2-hydroxyhexanoic acid

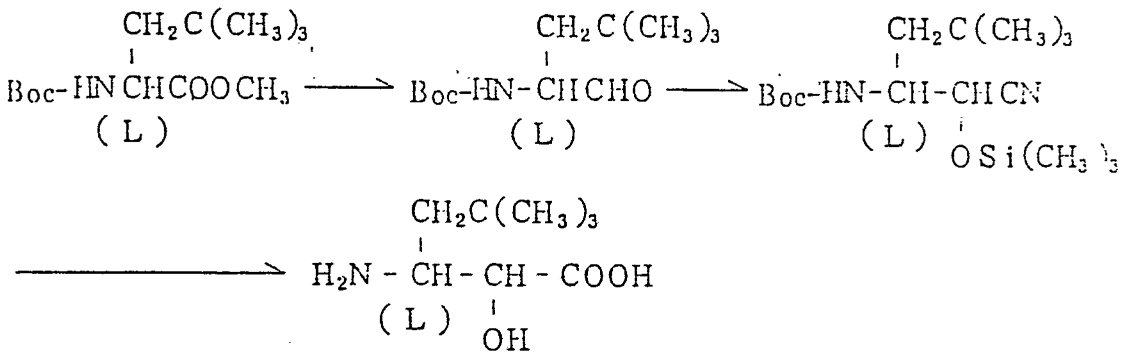

Reference Examples 34 to 36

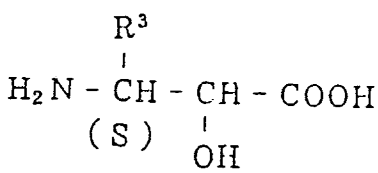

| | $R^3$ |
|---|---|
| 34. | 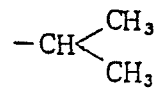 |
| 35. | 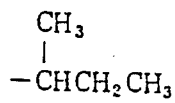 |

| | |
|---|---|
| 36. | $-(CH_2)_3CH_3$ |

Reference Example 37

(2RS,3S)-3-amino-5,5-dimethyl-2-hydroxyhexanoic acid iso-propyl ester

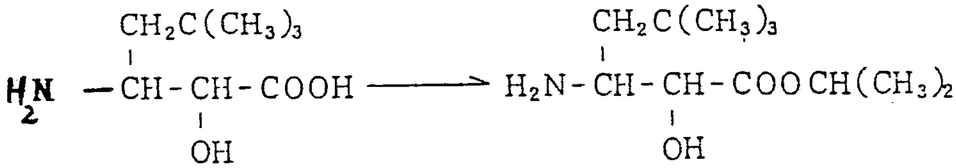

Reference Examples 38 to 44

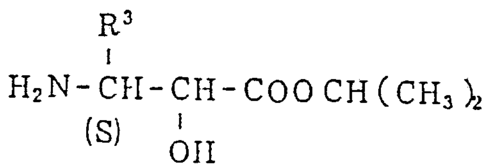

| | $R^3$ |
|---|---|
| 38. | 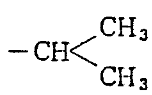 |

| | $R^3$ |
|---|---|
| 39. | $-CH(CH_3)CH_2CH_3$ |

| | $R^3$ |
|---|---|
| 40. | $-(CH_2)_3CH_3$ |
| 41. | $-(CH_2)_2CH_3$ |

42. (2RS,3S)-3-amino-2-hydroxy-4-cyclohexylbutyric acid
43. (2RS,3S)-3-amino-2-hydroxy-4-cyclohexylbutyric acid iso-propyl ester
44. (2RS,3S)-3-amino-2-hydroxy-4-cyclohexylbutyric acid methyl ester

## Reference Example 45

4-(p-fluorophenoxy)-2-(1-naphthylmethyl)butyric acid

## Reference Example 46

(2RS,3S)-3-N-t-butoxycarbonylamino-4-cyclohexyl-1-(1-methyl-5-tetrazolylthio)-2-butanol

1.4 ml of triethylamine (10 m mole) was added to a solution of 2.29 g (10 m mole) of 3-t-butoxycarbonyl-amino-4-cyclohexyl-1,2-oxobutane and 1.16 g (10 m mole) of 1-methyl-5-mercaptotetrazole in 20 ml of tetrahydrofuran, and the mixture was stirred overnight at room temperature. To the reaction mixture was added 100 ml of ethyl acetate, and the mixture was washed with water, dried over magnesium sulfate, was concentrated under reduced pressure. To the residue was added ether-n-hexane to give 1.5 g of white powder of the product compound.

Melting point: 129 - 136 °C

IR (KBr) $cm^{-1}$: 3356,2936,1688,1530,1178

NMR ($CDCl_3$): 1.42(s, 9H, t-BuO-), 3.38, 3.93

## Reference Examples 47 to 60

In similar manner to 46 above, the following were prepared:

47. (3S)-1-(benzothiazolylthio)-3-t-butyloxycarbonyl-amino)-1-(2-benzothiazolylthio)-4-cyclohexyl-2-butanol

48. (3S)-3-t-butyloxycarbonylamino-4-cyclohexyl-1-[(5-hydroxymethyl-1,3,4-thiadiazol-2-yl)-thio]-2-butanol

49. (3S)-3-t-butyloxycarbonylamino-4-cyclohexyl-1-[(5-methyl-1,3,4-thiadiazol-2-yl)thio]-2-butanol
50. (3S)-3-t-butoxycarbonylamino-4-cyclohexyl-1-(2-pyrimidinylthio)-2-butanol
51. (3S) -3-t-butoxycarbonylamino-4-cyclohexyl-1-(1,2,3-thiadiazol-5-ylthio)-2-butanol
52. (3S)-3-t-butoxycarbonylamino)-4-cyclohexyl-1-(1-ethyl-5-tetrazolyl)thio-2-butanol
53. (3S)-3-[N-t-butoxycarbonyl-L-histidyl)amino]-4-cyclohexyl-1-(2-thiazolylthio)-2-butanol
54. (3S)-3-[N-t-butoxycarbonyl-L-histidinyl]amino)-4-cyclohexyl-1-[(5-methyl-1,3,4-thiadiazol-2-yl)-thio]-2-butanol
55. (3S)-3-[N-t-butoxycarbonyl-L-histidyl)-amino)]-4-cyclohexyl-1-(2-pyrimidinylthio)-2-butanol
56. (3S)-3-[N-t-butoxycarbonyl-L-histidyl)amino]-4-cyclohexyl-1-(1,2,3-thiadiazol-5-ylthio)-2-butanol
57. (3S)-3-[(N-t-butoxycarbonyl-L-histidyl]amino)]-4-cyclohexyl-1-[(1-ethyl-5-tetrazolyl)thio]-2-butanol
58. (3S)-[(N-t-butoxycarbonyl-L-histidyl)]amino]-4-cyclohexyl-1-[(1-methyl-5-tetrazolyl)sulfonyl]-2-butanol
59. (3S)-N-t-butoxycarbonylamino)-4-cyclohexyl-1-[(1-methyl-5-tetrazolyl)sulfonyl]-2-butanol
60. (3S)-3-(N-t-butoxycarbonylamino)-5-methyl-1-nitro-2-hexanol

Example 1

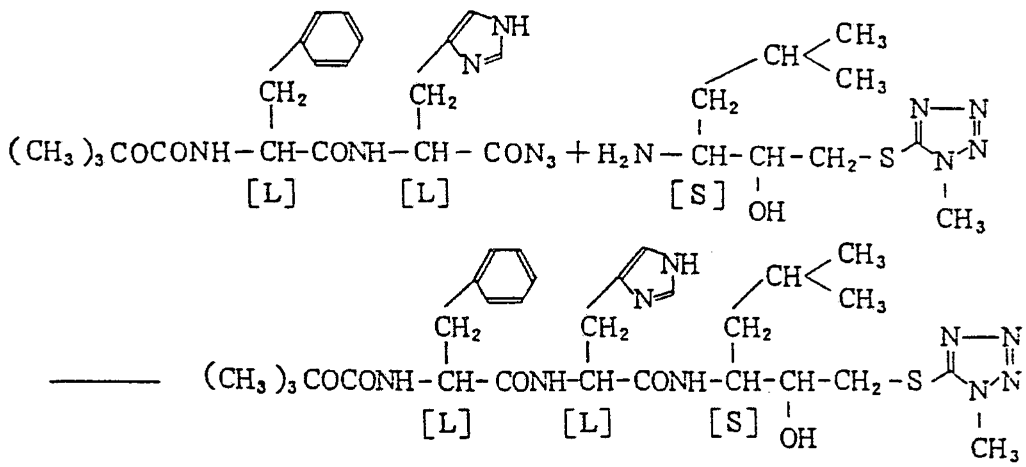

(2RS, 3S)-3-(N-t-butoxycarbonyl-L-phenylalanyl-L-histidylamino)-5-methyl-1-[(1-methyl-5-tetrazolyl)-thio]-2-hexanol

t-butoxycarbonyl-L-phenylalanyl-L-histidylhydrazide (416 mg, 1 m mole) was dissolved in 10 ml of dimethylformamide, and the obtained solution was cooled to -10 °C, and after adding thereto 0.84 ml of 4N/HCl/dioxane and 0.2 ml of iso-amyl nitrite while stirring, the mixture was stirred for 30 minutes at -30 °C, and 0.45 ml of N-methylmorpholine was added to the mixture while cooling at -40 °C.
On the other hand, 5 ml of 4N-HCl/dioxane was added to 345 mg of 3-N-t-butoxycarbonylamino-5-methyl-1-(1-methyl-5-tetrazolylthio)-2-hexanol, and the mixture was stirred for 1 hour at room temperature. The reaction solution was concentrated under reduced pressure to dryness, and the residue was dissolved in 10 ml of dimethylformamide. This solution was added to the above reaction mixture, and the obtained mixture was stirred overnight in an ice room.
To the reaction solution was added 100 ml of ethyl acetate, and after washing with aqueous $NaHCO_3$ and water, the mixture was dried over magnesium sulfate, and concentrated under reduced pressure. To the obtained residue was added chloroform, and the product compound was obtained as white powder (75 mg).
Rf = 0.17 (chloroform;methanol = 5:1)
Mass spectrum (m/z): 630 ($M^+$ + 1)
I.R. (KBr) $cm^{-1}$: 3312,1698,1660,1642

Example 2

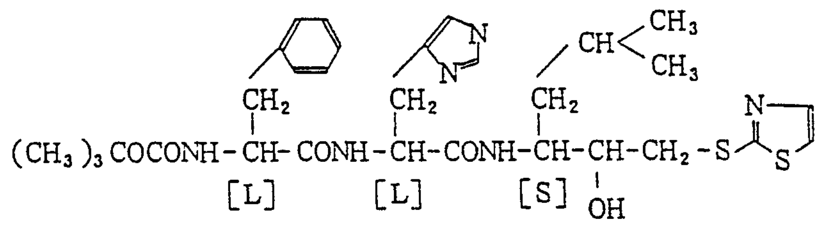

In similar manner to Example 1, (2RS,3S)-3-(N-t-butoxycarbonyl-L-phenylalanyl-L-histidylamino)-5-methyl-1-(2-thiazolylthio)-2-hexanol was obtained.
Rf = 0.58 (chloroform:methanol = 5:1)
Mass spectrum (m/z): 631 ($M^+$ + 1)
I.R. (KBr) $cm^{-1}$: 3316, 1698, 1644, 1537

Example 3

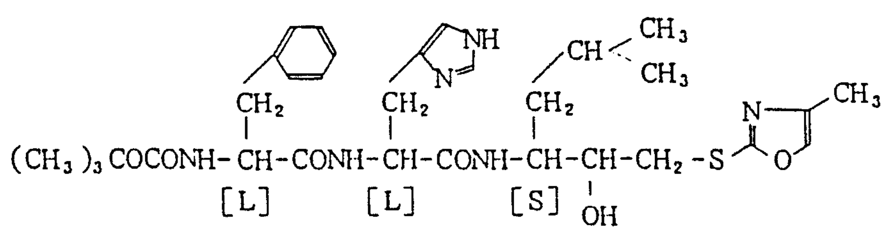

In similar manner to Example 1, (2RS,3S)-3-(N-t-butoxycarbonyl-L-phenylalanyl-L-histidylamino)-5-methyl-1-[4-methyloxazole-2-yl)thio]-2-hexanol was obtained.
Rf = 0.60 (chloroform:methanol:5:1)
Mass spectrum (m/z): 629 ($M^+$ + 1)
I.R. (KBr) $cm^{-1}$: 3328,1696,1646,1522,1170

Example 4

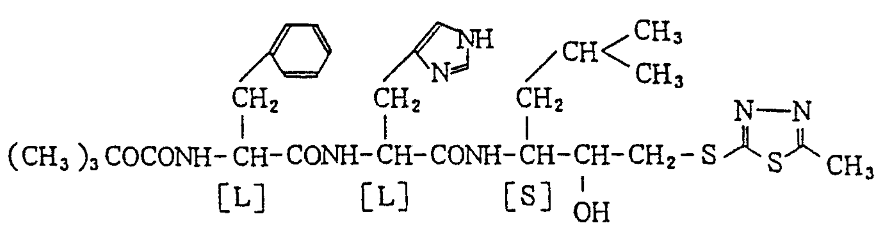

In similar manner to Example 1, (2RS,3S)-3-(N-t-butoxycarbonyl-L-phenylalanyl-L-histidylamino)-5-methyl-1-[(5-methyl-1,3,4-thiadiazol-2-ylthio]-2-hexanol was obtained.
Rf = 0.41 (chloroform:methanol = 5:1)
Mass spectrum (m/z): 646 ($M^+$ + 1)
I.R. (KBr) $cm^{-1}$: 3312,1698,1663,1642,1530

Example 5

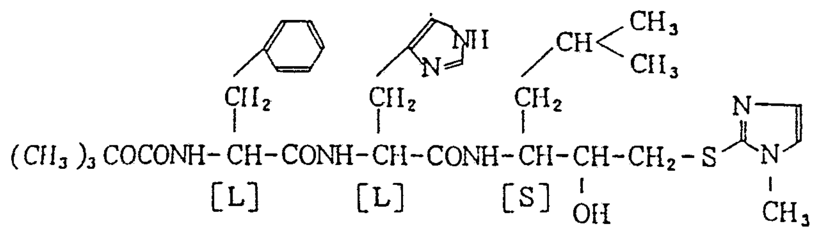

In similar manner to Example 1, (2RS,3S)-3-(N-t-butoxycarbonyl-L-phenylalanyl-L-histidylamino)-5-methyl-1-[(1-methylimidazol-2-yl)thio]-2-hexanol was obtained.
Rf = 0.55 (chloroform;methanol = 5:1)
Mass spectrum(m/z) : 628 ($M^+$ + 1)
I.R. (KBr) $cm^{-1}$: 3324,1654,1522

Example 6

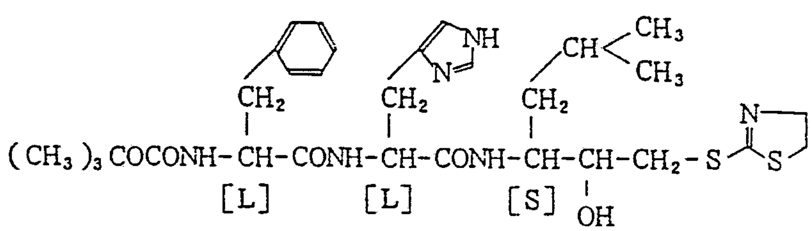

In similar manner to Example 1, was obtained (2RS,3S)-3-(N-t-butoxycarbonyl-L-phenylalanyl-L-histidylamino)-5-methyl-1-[2-thiazolin-2-yl)thio]-2-hexanol.
Rf = 0.62 (chloroform:methanol = 5:1)
Mass spectrum(m/z): 633($M^+$ + 1)
I.R. (KBr): $cm^{-1}$: 3328,1668,1558

Example 7

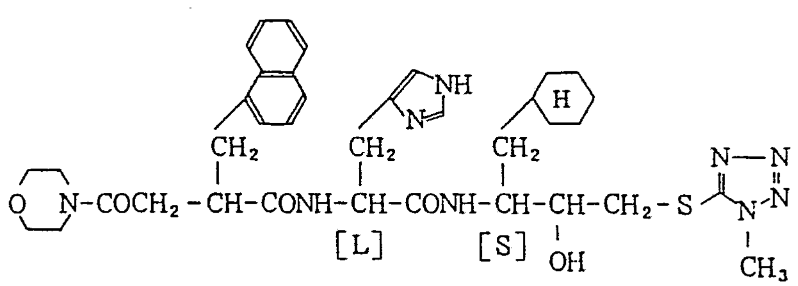

(2RS,3S)-3-[N-[1,4-dioxo-4-morpholino-2-(1-naphthyl-methyl)butyl-L-histidylamino)-4-cyclohexyl-1-(1-methyl-5-tetrazolylthio]-2-butanol

To 260 mg of (2RS,3S)-3-N-[(N-t-butoxycarbonyl-L-histidyl)amino]-4-cyclohexyl-1-(1-methyl-5-tetrazolylthio)-2-butanol (0.5 m mole), was added 5 ml of 4N-HCl/dioxane, and after stirring the mixture for 1 hour at room temperature, the mixture was concentrated under reduced pressure to dryness. To the residue was added 160 mg of 3-morpholinocarbonyl-2-(1-naphthyl-methyl)propionic acid and 5 ml of dimethylformamide and the mixture was stirred while ice-cooling. To the mixture was added 0.13 ml of diphenylphosphoryl azide and 0.21 ml of triethylamine, and the mixture was stirred overnight at room temperature. To the

reaction mixture was added 50 ml of ethyl acetate, and the mixture was washed with aqueous $NaHCO_3$ and water, dried over magnesium sulfate, and concentrated under reduced pressure. The residue was subjected to silica gel chromatography, and the fraction containing the product compound was concentrated. To the residue was added ether to give 70 mg of white powder of the product compound.
Rf = 0.50 (chloroform:methanol = 5:1)
Mass spectrum (m/z): 732 ($M^+$ + 1), 523
I.R. (KBr) $cm^{-1}$: 3328,1652,1450,1116

Example 8

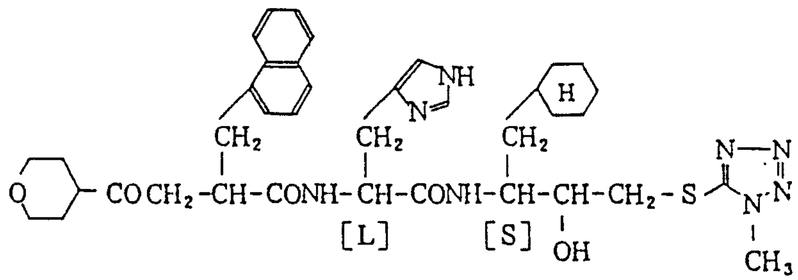

In similar manner to Example 1, was obtained (2RS,3S)-4-cyclohexyl-3-N-[N-[1,4-dioxo-2-(1-naphthylmethyl)-4-(tetrahydropyran-4-yl)butyl]-L-histidylamino]-1-(1-methyl-5-tetrazolylthio)-2-butanol.
Rf = 0.55 (chloroform:methanol = 5:1)
Mass spectrum (m/z): 731 ($M^+$ + 1)
I.R. (KBr) $cm^{-1}$: 3328,1705,1660

Example 9

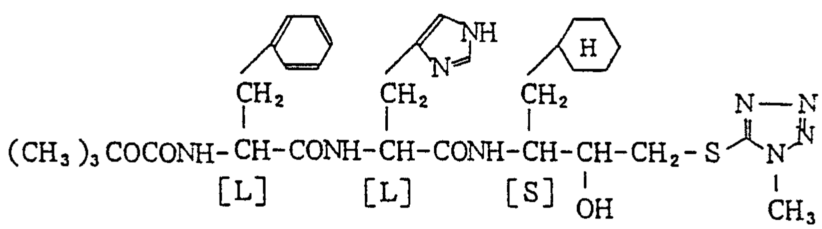

(2RS,3s)-3-(N-t-butoxycarbonyl-L-phenylalanyl-L-histidylamino)-4-cyclohexyl-1-(1-methyl-5-tetrazolyl-thio)-2-butanol.

A solution of 416 mg (1 m mole) of t-butoxycarbonyl-L-phenylalanyl-L-histidine hydrazide and 10 ml of dimethylformamide was cooled to -40°C, and after adding thereto 4N-HCl-dioxane (0.84 ml) and 0.2 ml of isoamyl nitrite, the mixture was stirred for 30 minutes at -20 to -30°C. After cooling the mixture to -40°C, N-methylmorpholine was added to the mixture.
On the other hand, to 385 mg of 3-(N-t-butoxycarbonyl-amino)-4-cyclohexyl-1-(1-methyl-5-tetrazolylthio)-2-butanol was added 5 ml of 4N-HCl/dioxane, and the mixture was stirred at room temperature overnight. The reaction solution was concentrated under reduced pressure, and the residue was dissolved in 10 ml of dimethylformamide. To this solution was added the above reaction solution, and the mixture was stirred in an ice-room overnight. To the reaction solution was added 100 ml of ethyl acetate and the mixture was washed with aqueous $NaHCO_3$ and water, dried over magnesium sulfate, and concentrated under reduced pressure. The residue was subjected to column chromatography, and the fraction containing the product compound was concentrated. To the residue was added ether to give 110 mg of white powder of the product compound.
Rf = 0.48 (chlroform:methanol = 5:1)
Mass spectrum (m/z): 670 ($M^+$ + 1)
I.R. (KBr) $cm^{-1}$: 3328,1696,1662,1642,1528,1172

Example 10

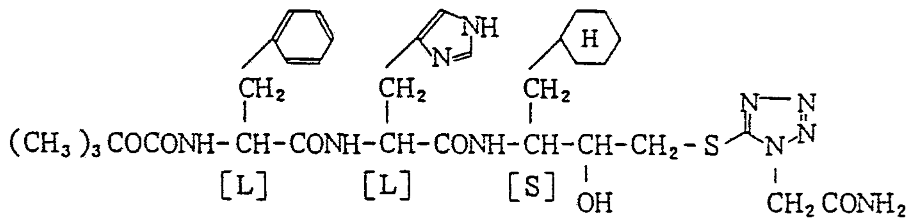

In similar manner to Example 9, was obtained (2RS,3S)-3-(N-t-butoxycarbonyl-L-phenylalanyl-L-histidylamino)-4-cyclohexyl-1-(1-carbamoylmethyl-5-tetrazolylthio)-2-butanol.
Rf = 0.27 (chloroform;methanol = 5:1)
Mass spectrum (m/z): 713 ($M^+ + 1$)
I.R. (KBr) $cm^{-1}$: 3328,1696,1664,1642,1170

Example 11

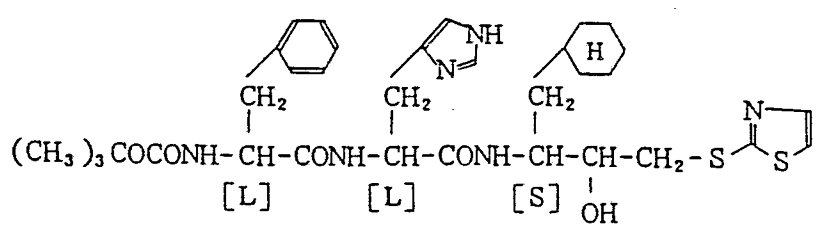

In similar manner to Example 9, was obtained (2RS,3S)-3-(N-butoxycarbonyl-L-phenylalanyl-L-histidylamino)-4-cyclohexyl-1-(2-thiazolylthio)-2-butanol.
Rf = 0.54 (chloroform:methanol = 5:1)
Mass spectrum (m/z): 671 ($M^+ + 1$)
I.R. (KBr) $cm^{-1}$: 3336,1696,1646,1528,1170

Example 12

(2RS,3S)-3-[N-[2(R,S)-t-butoxycarbonylamino-3-(1-naphthyl)propionyl]-L-histidyl]amino-2-hydroxy-4-cyclohexylbutyric acid iso-propyl ester

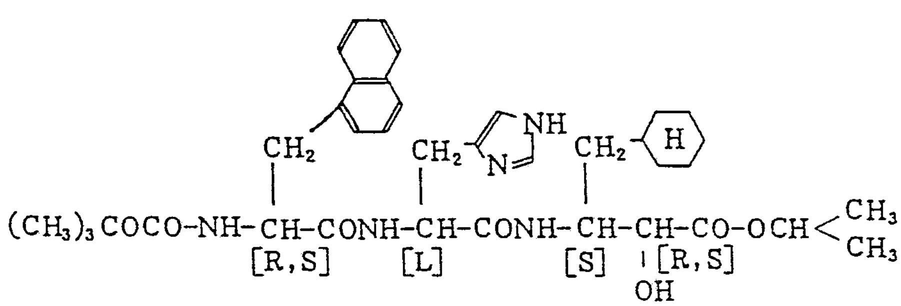

($R^1$: t-butoxycarbonylamino, $R^2$: iso-propoxy). A 4N-HCl/dioxane solution was added to 290 mg of N-Boc histidinyl-(2RS,3S)-3-amino-2-hydroxy-4-cyclochexyl butyric acid iso-propyl ester, and the mixture was stirred for 1 hour at room temperature. The reaction solution was concentrated under reduced pressure to dryness, and 190 mg of Boc-3-(1-naphthyl)alanine was added to the residue, and dissolved in dry dimethyl formamide, and after adding thereto 98 mg of cyanophosphoric acid diethyl ester and 122 mg of triethyl

amine under ice-cooling, the mixture was stirred for 30 minutes. The mixture was stirred overnight, and after adding thereto 50 ml of water, the mixture was extracted with 100 ml of ethyl acetate. The extract was wahsed with 5% $NaHCO_3$ aqueous solution and saturated NaCl aqueous solution, and dried over anhydrous magnesium sulfate. The solvent was removed by distillation under reduced pressure to give a colorless caramel of the crude product. This product was subjected to silica gel column chromatography (solvent: chloroform:methanol = 95:5) to purify it. Finally 170 mg. of colorless powder of the product compound was obtained.

Rf = 0.45 (chloroform:methanol = 9:1)

MS (FAB): 678 ($M^+$ + 1)

IR $^{KBR}_{cm^{-}}$ 1 ; 3280,2930,1710,1670

Examples 13 - 27

In similar manner to Example 12, the following compounds were obtained.

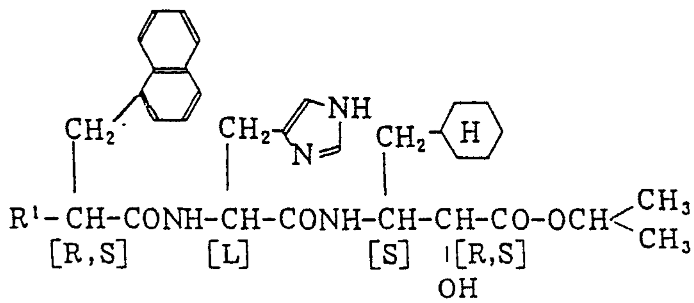

Example 13

(2RS,3S)-3-[N-[2-(1-naphthyl)methyl-3-(1-naphthyl)-propionyl]-L-histidyl]-amino-2-hydroxy-4-cyclohexyl butyric acid iso-propyl ester

( $R^1$ : 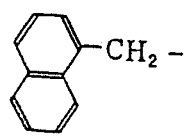 )

Example 14

(2RS,3S)-3-[N-[2-ethoxycarbonyl-3-(1-naphthyl)-propionyl]-L-histidyl]amino-2-hydroxy-4-cyclohexyl]butyric acid iso-propyl ester.

( $R^1$ : $CH_3CH_2OCO-$ )

Example 15

(2RS,3S)-3-[N-[2-(2-hydroxy-1(S)-methylethylcarbamoyl)-3-(1-naphthyl)propionyl]-L-histidyl]amino-2-hydroxy-4-cyclohexylbutyric acid isopropyl ester.

($R^1$: 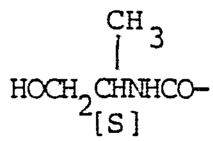 )

## Example 16

(2RS,3S)-3-[N-[(2RS)-4-(3-thienyl)-2-(1-naphthylmethyl) 4-oxobutanoyl]-L-histidyl]amino-2-hydroxy-4-cyclohexylbutyric acid iso-propyl ester.

($R^1$: 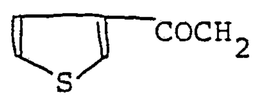 )

## Example 17

(2RS,3S)-3-[N-[(2RS)-4-(2-thienyl)-2-(1-naphthylmethyl) 4-oxobutanoyl]-L-histidyl]amino-2-hydroxy-4-cyclohexylbutyric acid iso-propyl ester.

( $R^1$ : 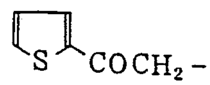 )

Rf = 0.59
MS (m/z) : 687 ($M^+$ +1)
IR$\nu_{max}^{KBr}$ $cm^{-1}$ : 2932, 1736, 1660

## Example 18

(2RS,3R)-3-[N-[(2RS)-2-(N-morpholinocarbamoyl)-2-(1-naphthyl)propionyl]-L-histidyl]amino-2-hydroxy-4-cyclohexylbutyric acid iso-propyl ester.

( $R^1$ : 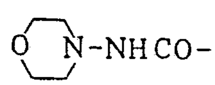 )

Rf : 0.45
MS (m/z) : 691 ($M^+$ +1)
IR$\nu_{max}^{KBr}$ $cm^{-1}$ : 2932, 1736, 1670

## Example 19

(2RS,3S)-3-[N-[2-(RS)-6-methyl-2-(1-naphthylmetyl)-4-oxohexnaoyl]-L-histidyl]amino-2-hydroxy-4-cyclohexyl-butyric acid iso-propyl ester.

( $R^1$ : 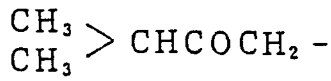 )

Rf = 0.54
MS (m/z) : 647 ($M^+$ +1 )
IR$\nu$ $^{KBr}_{max}$ cm$^{-1}$ : 2936, 1734, 1712, 1660

Example 20

(2RS,3S)-3-[N-2(RS)-4-(1,3-dioxolan-2-yl)-2-(1-naphthylmethyl)butanoyl[-L-histidyl]amino-2-hydroxy-4-cyclohexylbutyric acid iso-propyl ester.

( $R^1$ : 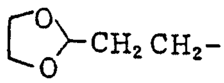 )

Rf = 0.67
MS (m/z) : 663, ($M^+$ +1)
IR$\nu$ $^{KBr}_{max}$ cm$^{-1}$ : 2936, 1734, 1652

Example 21

(2RS,3S)-3-[N-[2(RS)-4-(1,3-dioxan-2-yl)-2-(1-naphthylmethyl)butanoyl]-L-histidyl]amino-2-hydroxy-4-cyclohexylbutryic acid iso-propyl ester

( $R^1$ : 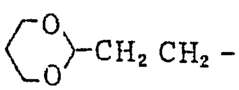 )

Rf = 0.45
MS (m/z) : 676 ($M^+$ +1)
IR$\nu$ $^{KBr}_{max}$ cm$^{-1}$ : 2936, 1736, 1654

Example 22

(2RS,3S)-3-[N-[2(RS)-4-phenyl-2-(1-naphthylmethyl)-4-oxobutanoyl]-L-histidyl]amino-2-hydroxy-4-cyclohexyl-butyric acid iso-propyl ester

( $R^1$ : 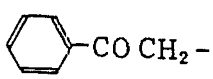 )

Rf = 0.63
MS (m/z) : 681 ($M^+$ +1)
IR$\nu$ $^{KBr}_{max}$ cm$^{-1}$ : 2932, 1736, 1682

Example 23

(2RS,3S)-3-[N-[(2RS)-2-(1-naphthylmethyl-3-(2-amino-thiazo-4-yl)propionyl]-L-histidyl]amino-2-hydroxy-4-cyclohexylbutyric acid iso-propyl ester.

( $R^1$ : 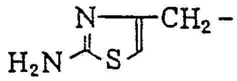 )

Rf = 0.34
MS (m/z) : 675 ($M^+$ + 1)
$IR\nu^{KBr}_{max}$ $cm^{-1}$ : 2988, 1732, 1654

Example 24

(2RS,3S)-3-[N-[(2RS)-2-(1-naphthylmethyl)-4-pentinoyl]-L-histidyl]amino-2-hydroxy-4-cyclohexylbutyric acid iso-propyl ester. ($R^1$ : $HC\equiv CCH_2$-)
Rf = 0.54
MS (m/z) : 601 ($M^+$ + 1)
$IR\nu^{KBr}_{max}$ $cm^{-1}$ : 3312, 1732, 1652

Example 25

(2RS,3s)-3-[[N-(2(RS)-3-cyano-2-(1-naphthylmethyl)-propionyl]-L-histidyl]amino-2-hydroxy-4-cyclohexylbutyric acid iso-propyl ester. ($R^1$ : $NCCH_2$-)
Rf = 0.63
MS (m/z) : 602 ($M^+$ + 1)
$IR\nu^{KBr}_{max}$ $cm^{-1}$ : 2936, 2253, 1738, 1664

Example 26

(2RS,3S)-4-cyclohexyl-3-[N-[4-cyclohexyl-1,4-dioxo-2-(1-naphthylmethyl)butyl]-L-histidyl]amino-2-hydroxybutyric acid iso-propyl ester.

( $R^1$ : 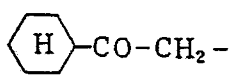 )

Rf = 0.17
MS (FAB) : (m/z) ; 687 ($M^+$ + 1)
$IR\nu^{KBr}_{max}$ $cm^{-1}$ : 2936, 2856, 1734, 1662

Example 27

(2RS,3S)-4-cyclohexyl-3-[N-[1,4-dioxo-2-(1-naphthyl-methyl)-4-(tetrahydropyran-4-yl)butyl]-L-histidyl]-amino-2-hydroxybutyric acid iso-propyl ester.

( $R^1$ : 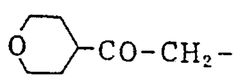 )

Rf = 0.08
MS (m/z) : 689 ($M^+$ + 1)
$IR\nu^{KBr}_{max}$ $cm^{-1}$ : 3336, 2936, 1734, 1664

Example 28

(2RS,3S)-4-cyclohexyl-2-hydroxy-3-[N-[3-(1-naphthyl-methyl)-2(R,S)-(2-phenetylaminocarbonyl)propionyl]-L-histidyl]aminobutyric acid iso-propyl ester.

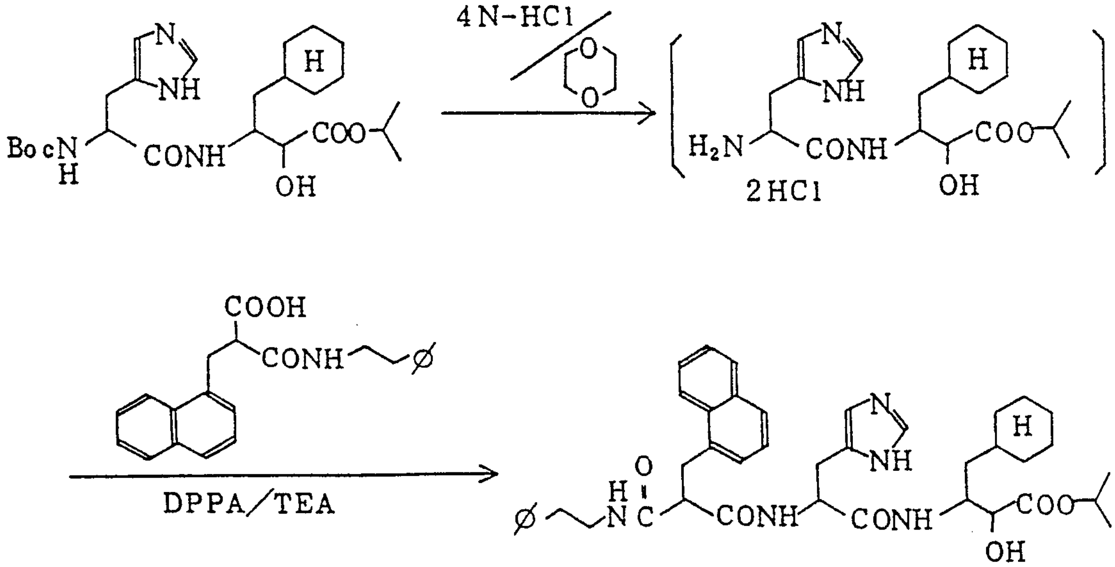


Rf = 0.36
MS : FAB (POS) (m/z) 710 ($M^+$ + 1), 181
IR $\nu_{max}^{KBr}$ $cm^{-1}$ : 3316, 2932, 2856, 1738

Example 29

(2RS,3S)-4-cyclohexyl-2-hydroxy-3-[N-[3-(1-naphthyl)-2-(R,S)-(1-naphthylaminocarbonyl)propionyl]-L-histidyl]-aminobutyric acid iso-propyl ester.

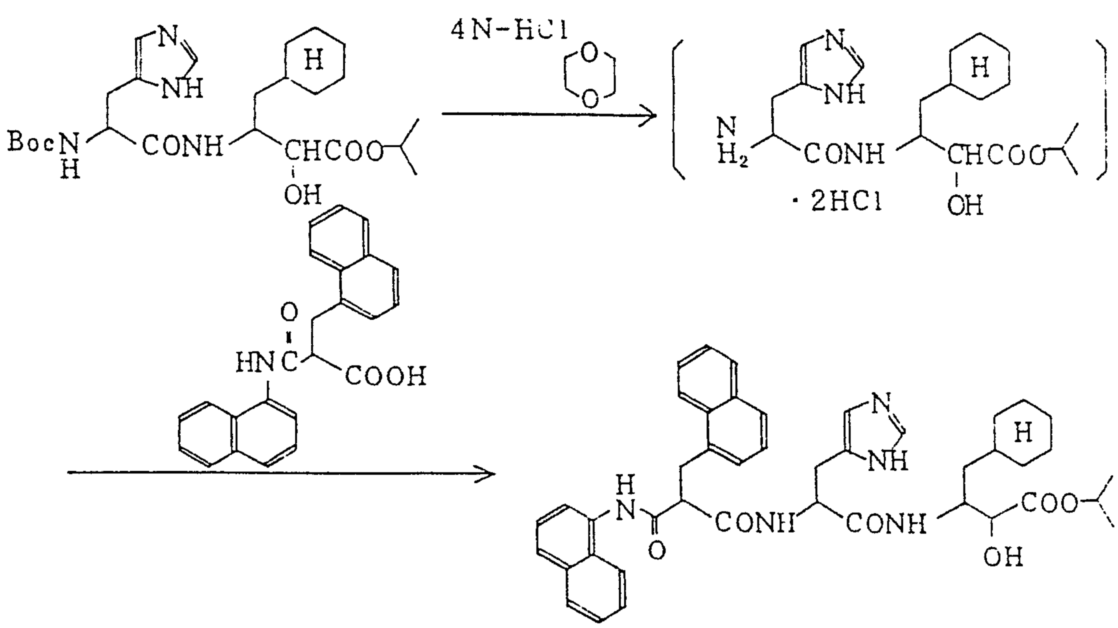


Rf = 0.40
MS ; FAB (pos) (m/z) 732 ($M^+$ + 1), 449
IR$\nu_{max}^{KBr}$ $cm^{-1}$ : 2932, 1732, 1670

Example 30

(2RS,3S)-4-cyclohexyl-2-hydroxy-3-[N-[2(R,S)-cyclo-hexylidenemethyl-3-(1-naphthyl)propionyl]-L-histidyl[-aminobutyric acid iso-propyl ester.

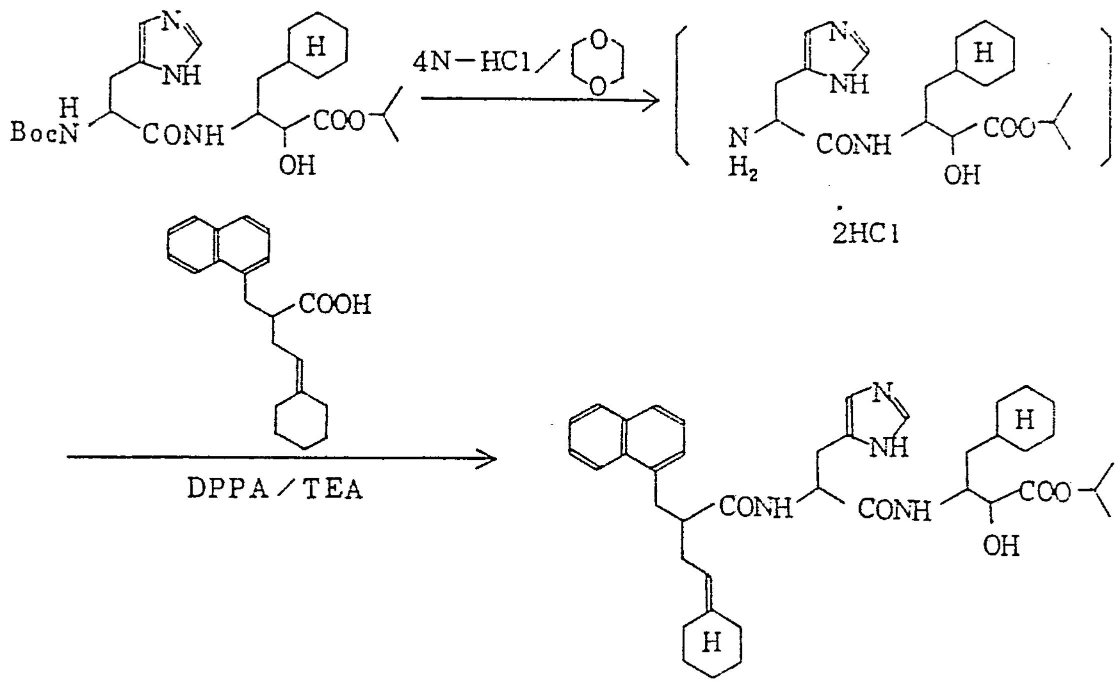


MS : FAB (pos);(m/z) 671 ($M^+$ +1), 400

IR$\nu_{max}^{KBr}$ $cm^{-1}$ : 3320, 2936, 2856, 1738

Example 31

(2RS,3S)-4-cyclohexyl-3-[N-[4-cyclohexyl-4-hydroxy-2-(1-naphthylmethyl-1-oxobutyl]-L-histidyl]amino-2-hydroxybutyric acid iso-propyl ester.

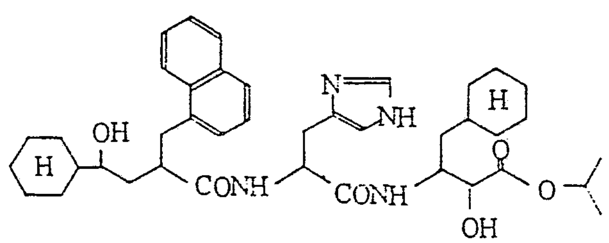

About 20 mg of sodium boron hydride was suspended in 3 ml of methanol, and the suspension was ice-cooled by ice-bath. To the suspension was added about 20 mg of (2RS,3S)-4-cyclohexyl-3-[N-[4-cyclohexyl-1,4-dioxo-2-(1-naphthylmethyl)butyl]-L-histidyl]amino-2-hydroxy-butyric acid iso-propyl ester, and the mixture was stirred for 40 minutes while ice-cooling.

To the reaction mixture was added 10 ml of saturated aqueous $NaHCO_3$, and the mixture was extracted with ethyl acetate (20 ml x 3). The organic layer was dried over anhydrous sodium sulfate, and the solvent was removed by distillation. The residue was dissolved in a small amount of ethyl acetate, and n-hexane was added thereto to give colorless crystals of the product. Yield: 17 mg.

Rf = 0.43(chloroform:methanol = 10:1)

MS : FAB(pos);(m.z) 689 ($M^+$ +1)

IR$\nu_{max}^{KBr}$ $cm^{-1}$: 3336, 2936, 1734, 1654

Example 32

In similar manner to Example 31, the following compound was obtained.

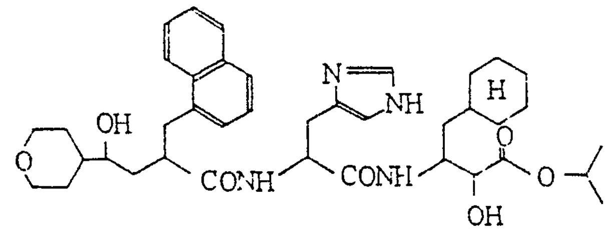

(2RS,3S)-4-cyclohexyl-2-hydroxy-3-[N-[4-hydroxy-2-(1-naphthylmethyl)-1-oxo-4-(tetrahydropyran-4-yl)butyl]-L-histidyl]aminobutyric acid iso-propyl ester.

Rf = 0.29 (chloroform:methanol = 10:1

MS (m/z) : 691 ($M^+$ + 1)

IR$\nu$ $^{KBr}_{max}$ $cm^{-1}$ : 3420, 2936, 1734, 1654

Example 33

(2RS,3S)-3-[N-[2-(1-naphthylmethyl)-3-(morpholino-carbonyl)propionyl]-L-histidyl]amino-2-hydroxy-hexanoic acid iso-propyl ester.

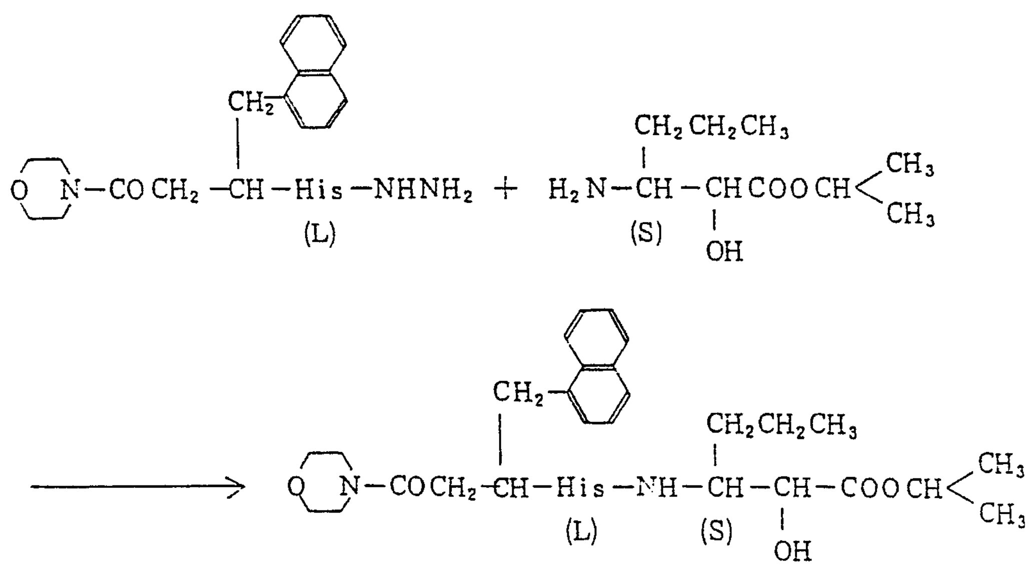

950 mg of N-[2-(1-naphthylmethyl)-3-(morpholino-carbonyl)propioyl]-L-histidine hydrazide is dissolved in 25 ml of dimethylformamide, and after adding thereto 1.6 ml of 4N HCl-dioxane (1.6 ml) and 0.46 ml of iso-amyl nitrite, the mixture was stirred for 30 minutes. The reaction mixture was cooled to -30°C, and neutralized with 0.92 ml of triethylamine to give a solution of N-[2-(1-naphthylmethyl)-3-(morpholinocarbonyl)propionyl]-L-histidine azide. This azide solution was added dropwise while cooling to a solution of 378 mg of (2RS,3S)-3-amino-2-hydroxy-hexanoic acid iso-propyl ester in 12 ml of dimethylformamide, and the obtained mixture was stirred overnight at 4 °C. To the reaction mixture was added aqueous saturated $NaHCO_3$, and the mixture was extracted with ethyl acetate. The organic layer was washed with saturated aqueous NaCl, and dried over anhydrous sodium sulfate. The solvent was removed by distillation under reduced pressure, and the residue was subjected to silica gel column chromatography, and eluted by chloroform;methanol (95:5) for purification. Finally 160 mg of colorless powder of the product compound was obtained.

Rf: 0.19 (chloroform:methanol = 95:5)

IR $^{KBr}_{max}$ $cm^{-1}$ ; 3290, 2960, 2930, 1730, 1640

MS (FAB) ; 636 ($M^+$ + 1)

Example 34

(2RS,3S)-3-[N-[2-(1-naphthylmethyl)-3-(morpholino-carbonyl)propionyl]-L-histidyl]amino-2-hydroxy-4-methylhexanoic aicd iso-propyl ester.

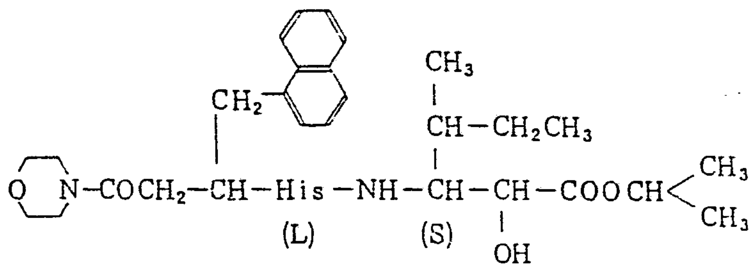

Using 624 mg of N-[2-(1-naphthylmethyl)-3-(morpholino-carbonyl)propionyl]-L-histidinehydrazide and 264 mg of (2RS,3S)-3-amino-2-hydroxy-4-methylhexanoic acid iso-propyl ester as starting materials, the reacton was carried out in similar manner to Examples 33, and the product was purified by silica gel column chromatography (eluting solution: chloroform-methanol = 95:5).

30 mg of the product compound was obtained

Rf: 0.29 (chloroform:methanol = 9:1)

IR $\nu_{max}^{KBr}$ $cm^{-1}$ ; 3300, 2970, 1730, 1640

MS (FAB) : 650 ($M^+$ + 1)

Examples 35 - 38

In similar manner to Example 33, the following compounds were obtained.

Example 35

(2RS,3S)-3-[N-[2-(1-naphthylmethyl-3-(morpholino-carbonyl)propionyl]-L-histidyl]amino-2-hydroxy-4-methylvaleric acid iso-propyl ester.

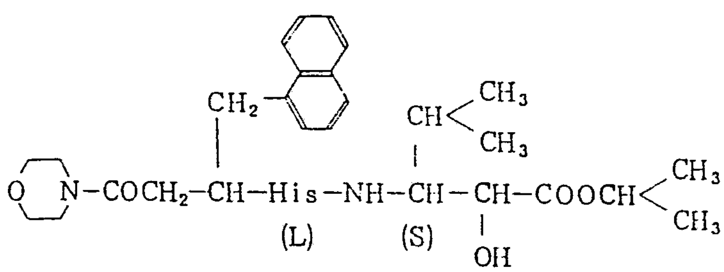

i) M.P. ; 100 ~ 101 °C

ii) Rf ; 0.21 (chloroform:methanol = 95:5)

iii) IR $\nu_{max}^{KBr}$ $cm^{-1}$ ; 3300, 2970, 1730, 1660, 1630

MS (FAB) ; 636 ($M^+$ + 1)

Example 36

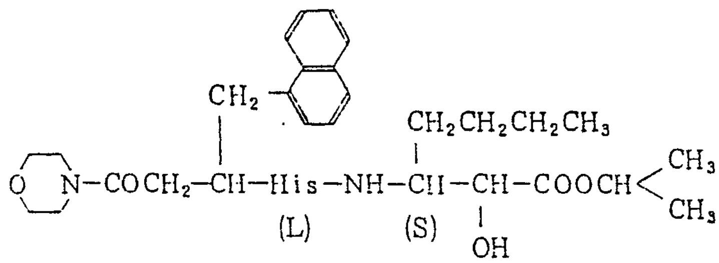

(2RS,3S)-3-[N-[2-(1-naphthylmethyl)-3-(morpholino-carbonyl)propionyl]-L-histidyl]amino-2-hydroxy-heptanoic acid iso-propyl ester. M.P. ; 85 ~ 87 °C

Rf ; 0.18 (chloroform:methanol = 95:5)

IR $^{KBr}_{max}$ $cm^{-1}$ ; 3290, 2940, 1735, 1640

MS (FAS) ; 650 ($M^{+}$ + 1)

Example 37

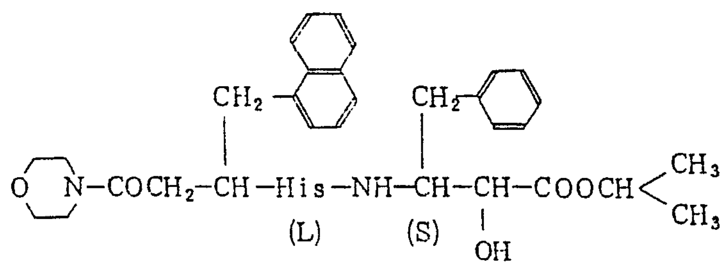

(2RS,3S)-3-[N-[2-(1-naphthylmethyl-3-(morpholino-carbonyl)propionyl]-L-histidyl]amino-2-hydroxy-4-phenylbutyric acid iso-propyl ester. IR $^{KBr}_{max}$ $cm^{-1}$ ; 1738, 1646, 1112

MS (FAB) ; 684 ($M^{+}$ + 1)

Example 38

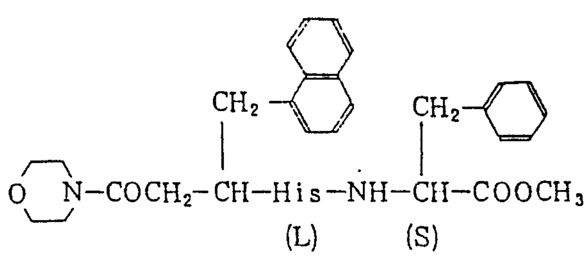

(2RS,3S)-3-[N-[2-(1-naphthylmethyl)-3-(morpholino-carbonyl)propionyl]-L-histidyl]amino-2-hydroxy-4-phenylbutyric acid methyl ester. IR $^{KBr}_{max}$ $cm^{-1}$ ; 1748, 1646, 1116

MS (FAS) ; 656 ($M^{+}$ + 1)

Example 39

(2RS,3S)-3-[n-[4-(p-fluorophenoxy)-2-(1-naphthylmethyl)-butyryl]-L-histidyl]amino-2-hydroxy-4-cyclohexyl-butyric acid iso-propyl ester.

(a) N-[4-(p-fluorophenoxy)-2-(1-naphthylmethyl)butyryl]-L-histidine methyl ester.

20 ml of dimethylformamide was added to 1.5 g of 4-(p-fluorophenoxy)-2-(1-naphthylmethyl)butyric acid 0.6 g of 1-hydroxybenzotriazole and 1.1 g of L-histidine 2HCl salt, and the mixture was neutralized with 1.3 ml of triethylamine, and after adding thereto 1.0 g of N,N'-dicyclohexylcarbodiimide, the mixture was stirred overnight at room temperature. The insoluble matter was removed, and dimethylformamide was removed by distillation under reduced pressure. To the residue was added 100 ml of methylene chloride, and the mixture was washed with saturated aqueous $NaHCO_3$ and water, and the organic layer was dried over anhydrous magnesium sulfate followed by being subjected to silica gel column chromatography for purification. Finally 1.7 g of white powder of the product compound was obtained.
Melting point: 65 - 69 °C
IR ($cm^{-1}$): 1746,1656,1508,12250
MS: 490 ($M^+$ +1)

(b) N-[4-(p-fluorophenoxy)-2-(1-naphthylmethyl)butyryl]-L-histidine hydrazide.

1.7 g of N-[4-p-fluorophenoxy)-2-(1-naphthylmethyl)-butyryl]-L-histidine obtained in 39 (a) was dissolved in 40 ml of methanol, and after adding thereto 0.6 ml of hydrazine hydrate, the mixture was stirred overnight at room temperature. The reaction solution was concentrated under reduced pressure, and after adding to the residue 100 ml of ethyl acetate, the mixture was washed with water, dried ovre magnesium sulfate, and concentrated under reduced pressure to give white powder of the product compound (1.5g).
Melting point: 87 - 91 °C
IR ($cm^{-1}$): 3292,1654,1508,1250

(c) (2RS,3S)-3-[N-[4-(p-fluorophenoxy)-2-(1-naphthyl-methyl)butyrl]-L-histidyl]amino-2-hydroxy-4-cyclohexylbutyric acid iso-propyl ester.

489 mg of N-[(p-fluorophenoxy)-2-(1-naphthylmethyl)-butyryl]-L-histidine hydrazide was dissolved in 18 ml of dimethylformamide, and after adding thereto, at -40 °C, 0.84 ml of 4N HCl-dioxane and then 0.2 ml of iso-amyl nitrite, the mixture was stirred for 30 minutes at -20 ° to -30 °C. The reaction mixture was cooled to -40 °C, and after adding thereto 0.34 ml of N-methylmorpholine and then 243 mg of (2RS,3S)-3-amino-2-hydroxy-4-cyclohexylbutyric acid iso-propyl ester, the reaction mixture was stirred at 0 - 5 °C overnight. The reaction mixture was poured into 100 ml of ice-water, extracted with methylene chloride, and washed with saturated $NaHCO_3$ aqueous solution and water, and dried over anhydrous magnesium sulfate followed by silica gel column chromatography for purification. Finally 300 mg of white powder of the product compound (Rf 0.52) was obtained.
Rf value: thin layer column chromatography (Merck, pre-coat plate silica gel $60F_{254}$), eluting solution (chloroform-methanol = 5:1).

Example 40

(2RS,3S)-3-[N-[2-(1-naphthylmethyl)-3-morpholinocarbonyl)-propionyl]-L-histidyl]amino-2-hydroxy-4-cyclohexylbutyric acid iso-propyl ester.

480 mg of N-[2-(1-naphthylmethyl)-3-(morpholinocarbonyl)-propionyl]-L-histidine hydrazide was dissolved in 13 ml of dimethylformamide, and after adding thereto, at -20°C, 0.8 ml of 4N HCl-dioxane and 0.17 ml of iso-amyl nitrite, the mixture was stirred for 30 minutes. The reaction mixture was cooled to -30 °C, and neutralized with triethylamine to give a solution of N-[2-(1-naphthyl-methyl)-3-(morpholinocarbonyl)-propionyl]-L-histidine azide. This azide solution was added dropwise under ice-cooling to a solution of 243 mg of (2RS,3S)-3-amino-2-hydroxy-4-cyclohexylbutyric acid iso-propyl ester in 25 ml of dimethylformamide, and the mixture was stirred overnight at 4 °C. To the reaction solution was added saturated aqueous $NaHCO_3$ and the mixture was extracted with ethyl acetate. The organic layer was washed with saturated aqueous NaCl, and dried over anhydrous sodium sulfate. The solvent was removed by distillation under reduced pressure, and the residue was subjected to silica gel column chromatography, and eluted by

chloroform-methanol (95:5) for purification Finally 350 mg of colorless powder of the product compound (Rf = 0.34) (350 mg) was obtained.
Melting point: 103 - 107 °C
MA(FAB): 690 ($M^+$ + 1)

Example 41

(2RS,3s)-3-[N-[2-(1-naphthylmethyl)-3-(morpholino-carbonyl)propionyl]-L-histidyl]amino-2-hydroxy-4-cyclohexylbutyric acid methyl ester.

Using N-[2-(1-naphythylmethyl)-3-(morpholinocarbonyl)-propionyl]-L-histidine hydrazide (480 mg) and 243 mg of (2RS,3S)-3-amino-2-hydroxy-4-cyclohexylbutyric acid methyl ester, the reaction was carried out in similar manner to Example 40, and the product was purified by silica gel column chromatography (eluting solution: chloroform-methanol = 95:5). Finally 150 mg of colorless powder of the product compound (Rf = 0.64) was obtained.
Melting point: 83 - 87 °C
MS (FAB): 662 ($M^+$ + 1)

Example 42

N-(2-benzyl-3-phenylpropionyl)-L-histidyl-(2RS,3S)-3-amino-2-hydroxy-4-cyclohexylbutyric acid iso-propyl ester/

(a) N-tert-butoxycarbonyl-L-histidyl-(2RS,3S)-3-amino-2-hydroxy-4-cyclohexylbutyric acid iso-propyl ester.

2.44 g of (2RS,3S)-3-amino-2-hydroxy-4-cyclohexyl-butyric acid iso-propyl ester and 2.75 g of N-tert-butoxycarbonyl-L-histidine was dissolved in 130 ml of dimethylformamide, and after adding thereto, under ice-cooling, 1.85 g of cyanophosphoric acid diethyl ester and 2.17 g of triethylamine, the mixture was stirred for 30 minutes under ice-cooling, and then at room temperature for 4 hours. The solvent was removed by distillation under reduced pressure, and water was added to the residue. The mixture was extracted with ethyl acetate and the organic layer was washed with 10 % aqueous citric acid, saturated $NaHCO_3$ aqueous solution and saturated aqueous NaCl, and dried over anhydrous sodium sulfate. The solvent was removed by distillation under reduced pressure, and the residue was subjected to column chromatography (silica gel), and eluted by chloroform-methanol (97:3) for purification Finally powder of the product compound (700 mg) was obtained.
NMR ($CDCl_3$) $\delta$ (ppm) : 0.6 -2.0 (m, 33H), 2.9-3.1(m,1H) 4.08(d, 1H, J = 3.1Hz), 4.1-4.6(m, 1H), 4.9-5.2(m, 1H) 5.6-5.9(m, 1H), 6.84 (s, 1H), 7.56(s,1H)
MS: 480($M^+$)

(b) N-(2-benzyl-3-phenylpropionyl)-L-histidyl-(2RS,3S)-3-amino-2-hydroxy-4-cyclohexylbutyric acid iso-propyl ester.

120 mg of N-tert-butoxycarbonyl-L-histidyl-(2RS,3S)-3-amino-2-hydroxy-4-cyclohexylbutyric acid iso-propyl ester was dissolved in 60 ml of 4N HCl-dioxane, and the mixture was stirred for 40 minutes at room temperature. The solvent was removed by distillation under reduced pressure to dryness, and after adding thereto 60 mg of 2-benzyl-3-phenylpropionic acid, the mixture was dissolved in 3 ml of dimethylformamide. After adding thereto, under ice-cooling, 41 mg of cyanophosphoric acid diethyl ester and then 53 mg of triethylamine, dropwise, the mixture was stirred for 30 minutes under ice-cooling, and then overnight at room temperature. The solvent was removed by distillation, and water was added to the residue. The obtained mixture was extracted with ethyl acetate, and the organic layer was washed with 5% HCl, saturated $NaHCO_3$ aqueous solution and then saturated aqueous NaCl, and dried over anhydroux magnesium sulfate. The solvent was removed by distillation under reduced pressure, and the residue was

subjected to column chlomatography (silica gel) and eluted with chloroform-methanol (95:5) for purification. Finally 70 mg of the product compound was obtained as colorless powder (Rf = 0.61).
Melting point: 91 = 94 °C
MS(FAB): 603 ($M^+$ + 1)

Example 43

In similar manner to Example 1, (3S)-3-(N-t-butoxy-carbonyl-L-phenylalanyl-L-histidylamino)-5-methyl-1-nitro-2-hexanol was obtained.

Examples 44-51

In similar manner to Example 8, the following compounds were prepared :
(3S)-4-cyclohexyl-3-[(N-[1,4-dioxo-4-morpholino-2-(1-naphthylmethyl)butyl]-L-histidyl]amino]-1-(2-thiazolylthio)-2-butanol;
(3S)-4-cyclohexyl-3-[(N-[1,4-dioxo-4-morpholino-2-(1-naphthylmethyl)butyl]-L-histidyl]amino]-1-[(5-methyl-1,3,4-thiadiazol-2-yl)thio]-2-butanol;
(3S)-4-cyclohexyl-3-[(N-[1,4-dioxo-4-morpholino-2-(1-naphthylmethyl)butyl]-L-histidinyl]amino] -1-(2-pyrimidinylthio)-2-butanol;
(3S)-4-cyclohexyl-3-[(N-[1,4-dioxo-4-morpholino-2-(1-naphthylmethyl)butyl]-L-histidyl]amino]-1-(1,2,3-thiadiazol-5-ylthio]-2-butanol;
(3S)-cyclohexyl-3-[(N-[1,4-dioxy-2-(1-naphthylmethyl)-4-(3-thienyl)butyl]-L-histidyl]amino]-1-(1-methyl-5-tetrazolyl)-2-butanol;
(3S)-4-cyclohexyl-3-[(N-[[1,4-dioxo-4-morpholino)-2-(1-naphthyl)butyl]-L-histidyl]amino]-1-[(1-ethyl-5-tetrazolyl)thio]-2-butanol;
(3S)-4-cyclohexyl-3-[(N-[1,4-dioxo-4-morpholino)-2-(1-naphthylmethyl)butyl]-L-histidyl]amino]-1-[(1-methyl-5-tetrazolylsulfonyl]-2-butanol;
(3S)-4-cyclohexyl-3-[(N-[1,4-dioxo-4-morpholino)-2-(1-naphthylmethyl)butyl]-L-histidyl]amino]-1-nitro-2-butanol

Examples 52 - 54

In similar manner to Example 9, the following were prepared.
(3S)-1-(2-benzthiazolylthio)-3-[(N-t-butoxycarbonyl-L-phenylalanyl-L-histidyl)amino]-4-cyclohexyl-2-butanol
(3S)-3-[(N-t-butoxycarbonyl-L-phenylalanyl-L-histidyl)-amino)]-4-cyclohexyl-1-[(5-hydroxymethyl-1,3,4-thiadia-zol-2-yl)thio]-2-butanol;
(3S)-3-((N-butoxycarbonyl-L-phenylalanyl-L-histidyl)amino)]-4-cylcohexyl-1-nitro-2-butanol.

Example 55

(3S)-3-[N-(t-butyloxycarbonyl-1-phenylalanyl-L-histidyl)-N-methyl]amino-4-cyclohexyl-2-hydroxy-butanoic acid iso-propyl ester.

0.21 g of t-butyloxycarbonyl-L-phenylalanyl-L-histidyl-hydrazide was dissolved in 3 ml of dimethylformamide, and after cooling the mixture to -60°C and adding thereto 0.43ml of 4N.HCl/1,4-dioxane and 0.10ml of isoamyl nitrite, the mixture was stirred for 30 minutes at -20°C. The reaction mixture was cooled to -60°C, and after adding 0.30ml of N-methylmorpholine and then 5ml of dimethylformamide solution containing (3S)-4-cyclohexyl-2-hydroxy-3-methylaminobutanoic acid iso-propyl ester HCl salt, the mixture was stirred for 1 day at 4°C. To the reaction solution was added 50ml of saturated aqueous $NaHCO_3$, and the mixture wsa extracted with ethyl acetate (100ml x 3). The organic layer was washed with 50ml of water and saturated aqueous NaCl, successively, and then was dried over anhydrous sodium sulfate, and the

solvent was removed by distillation. The residue was subjected to column chromatography (silica gal) (chloroform;methanol = 100:1 - 10:1) to purify the product. By treating with ether-n-hexane, 0.12g of white powder of the product compound was obtained.
Rf: 0.39 (chloroform;methanol = 10:1)

Example 56

(3S)-3-[[N-[3-(morpholino)carbonyl)-2-(1-naphthylmethyl)-propionyl]-L-histidyl]-N-methyl]amino)-4-cyclohexyl-2-hydroxybutanoic acid iso-propyl ester.

100mg of (3S)-3-[N-(t-butyloxycarbonyl-L-histidyl)-N-methyl]amino-4-cyclohexyl-2-hydroxybutanoic acid iso-propyl ester was dissolved in 3ml of 4N-HCl/dioxane, and the solution was stirred for 1 hour at room temperature. The solvent was removed by distillation, and the residue was dissolved in 3ml of dimethylformamide. The solution was cooled in ice, and after adding thereto 0.11 ml of triethylamine, 76mg of 3-(morpholinocarbonyl)-2-(1-naphtylmethyl)propionic acid and 0.05 ml of dipheyl-phosphatidyl azide, the mixture was stirred for 1 day at room temperature. To the mixture was added 30 ml of saturated aqueous $NaHCO_3$, and the mixture was extracted with ethyl acetate (50ml X 3), and the organic layer was washed with 30ml of water, dried over anhydrous sodium sulfate, and then the solvent was removed by distillation. The residue was subjected to silica gel column chromatography (chloroform;methanol = 100:1 - 20:1) to purify the product. By treating with ether-n-hexane, 50 mg of white powder of the product compound was obtained.
Rf;0.44 (chloroform;methanol = 10:1)

Example 57

Iso-propyl(3S)-3-(t-butyloxycarbonyl-L-phenylalanyl-L-histidyl)amino-2-cyclohexyl-2-hydroxybutyrate. For this example, 0.48 g of iso-propyl (3S)-3-(t-butyloxycarbonyl-L-histidyl)-amino-2-cyclohexyl-2-hydroxybutyrate was dissolved in 10 ml of 4N HCl-dioxane, and after stirring the solution at room temperature for 1 hour, the solvent was removed by distillation, and the residue was dissolved in 4 ml of dimethylformamide. After cooling the solution with ice, 0.52 ml of triethylamine, 0.29 g of t-butyloxycarbonylphenylalanine, and 0.26 g of diphenylphosphatidylazide were added successively to the solution. After stirring the mixture at room temperature, 30 ml of saturated aqueous $NaHCO_3$ was added thereto, and the obtained mixture was extracted with ethyl acetate (50 ml x 3). The organic layer was washed wiht 30 ml of water, dried over anhydrous sodium sulfate, and the solvent was removed by distillation. The residue was subjected silica-gel column chromatography ($CHCl_3$ : methanol = 100:1 = 10:1) to purify the product. The product was crystallized from ether-n-hexane to give white powder of the product compound (505 mg).

Rf: 0.33 (chloroform:methanol = 10:1)
MS (FAB): 628 ($M^+ + 1$)
IR (KBr) ($cm^{-1}$): 3432, 2936, 1724, 1662

Reference Example 61

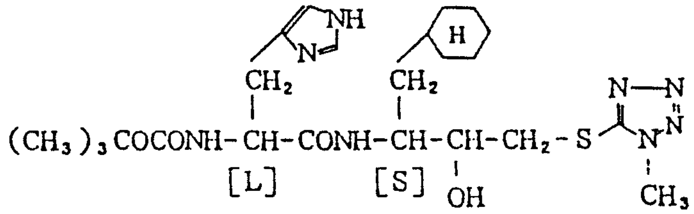

(2RS,3S)-3-N-[(N-1-butoxycarbonyl-L-histidyl)amino-4-cyclohexyl-1-(1-methyl-5-tetrazolylthio)-2-butanol

To 1 g of 3-N-t-butoxycarbonylamino-4-cyclohexyl-1-(1-methyl-5-tetrazolylthio)-2-butanol (2.6 mmole) was added 15 ml of 4N-HCl/dioxane, and after stirring the mixture at room temperature for 1 hour, the mixture was concentrated under reduced pressure to dryness. To the residue was added 663 mg of N-t-butoxycarbonyl-L-histidine and 350 mg of 1-hydroxybenzotriazole and 20 ml of dimethylformamide. The mixture was stirred under ice-cooling, and after adding thereto 0.37 ml of triethyl-amine and 600 mg of N,N'-dicyclohexylcarbodiimide, the mixture was stirred at room temperature overnight. The insoluble matter was removed by filtration, and after adding thereto 100 ml of ethyl acetate, the mixture was washed with aqueous $NaHCO_3$ and water, dried over magnesium sulfate, and concentrated. To the residue was added ether-n-hexane and 520 mg of white powder of the product compound was obtained.
Rf = 0.45 (chloroform methanol = 5:1)
MS(m/z):523($M^+$ + 1), 423
IR(KBr) $cm^{-1}$:3364,2936,1698,1646,1526,1172

Example 58

In similar manner to Ex.33, (2RS,3S)-3-[N-[2-(1-naphthyl-methyl)-3-morpholinocarbonyl)propionyl]-L-histidyl]amino-5,5-dimethyl-2-hydroxyhexanoic acid iso-propyl ester was obtained.
Melting point:101-104°C

The following Table summarises preferred compounds of the invention from the foregoing Examples.

# TABLE

| $R_1$ | $R_2$ | $R_3$ | $R_4$ |
|---|---|---|---|
| 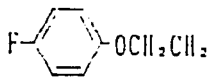 | 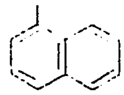 | 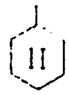 | 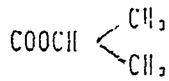 |
| 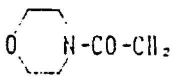 | 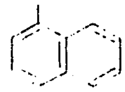 | 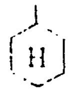 | 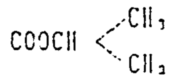 |
| 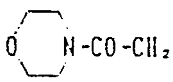 | 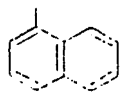 | 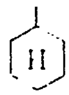 | $COOCH_3$ |
| 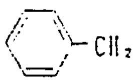 | 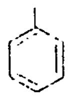 | 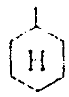 | 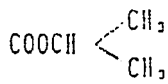 |
| 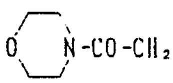 | 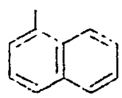 | $-CH_2CH_3$ | 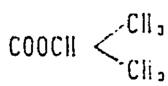 |
| 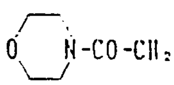 | 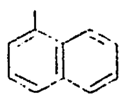 | $( -CH_2-R_3 )$ 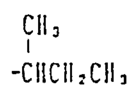 | 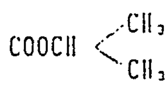 |
| 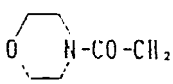 | 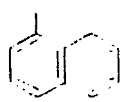 | $( -CH_2-R_3 )$ 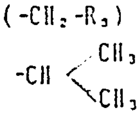 | 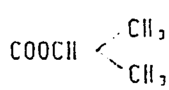 |
| 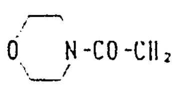 | 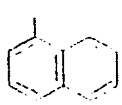 | $-CH_2CH_2CH_3$ | 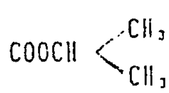 |

| | | | |
|---|---|---|---|
| 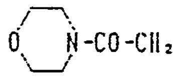 | 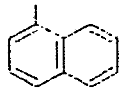 | 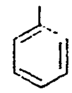 | 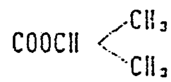 |
| 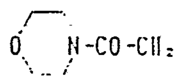 | 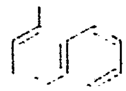 | 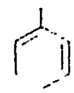 | $-COOCH_3$ |
| $(CH_3)_3COCONH$ | 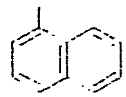 | 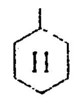 | 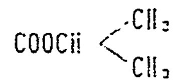 |
| 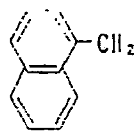 | 〃 | 〃 | 〃 |
| $CH_3CH_2OCO-$ | 〃 | 〃 | 〃 |
| $CH_3$<br>\|<br>$HOCH_2CHNHCO-$ | 〃 | 〃 | 〃 |
| 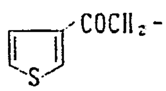 | 〃 | 〃 | 〃 |
| 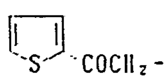 | 〃 | 〃 | 〃 |

| | | | |
|---|---|---|---|
| 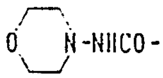 | 〃 | 〃 | 〃 |
| 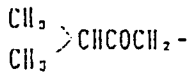 | 〃 | 〃 | 〃 |
| 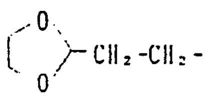 | 〃 | 〃 | 〃 |
| 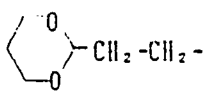 | 〃 | 〃 | 〃 |
| 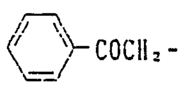 | 〃 | 〃 | 〃 |
| 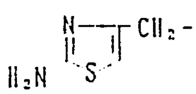 | 〃 | 〃 | 〃 |
| $HC \equiv C-CH_2-$ | 〃 | 〃 | 〃 |
| $NCCH_2-$ | 〃 | 〃 | 〃 |

| | | | |
|---|---|---|---|
| 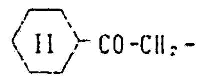 | 〃 | 〃 | 〃 |
| 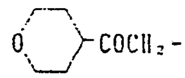 | 〃 | 〃 | 〃 |
| 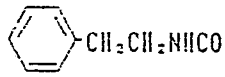 | 〃 | 〃 | 〃 |
| 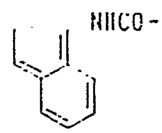 | 〃 | 〃 | 〃 |
| 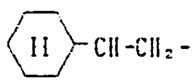 | 〃 | 〃 | 〃 |
| 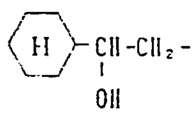 | 〃 | 〃 | 〃 |
| $(CH_3)_3COCONH-$ | 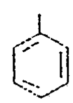 | 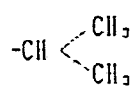 | $CH_2$-S-TZ |
| $(CH_3)_3COCONH-$ | 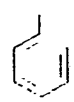 | 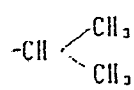 | 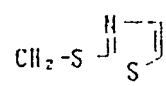 |

| | | | |
|---|---|---|---|
| $(CH_3)_3COCONH$ | 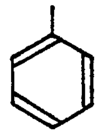 | 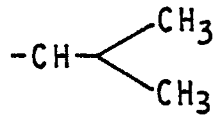 | 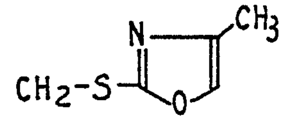 |
| $(CH_3)_3COCONH$ | 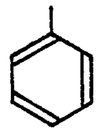 | 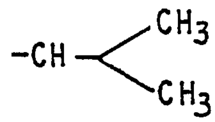 | 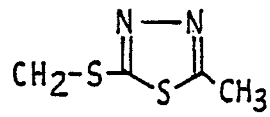 |
| $(CH_3)_3COCONH$ | 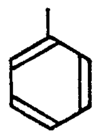 | 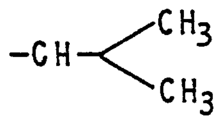 | 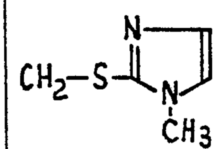 |
| $(CH_3)_3COCONH$ | 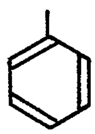 | 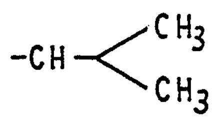 | 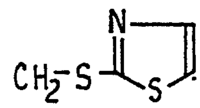 |
| 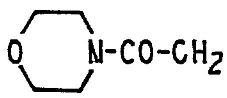 | 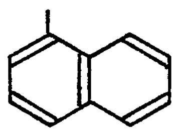 | 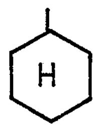 | $CH_2-S-TZ$ |
| 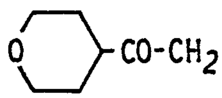 | 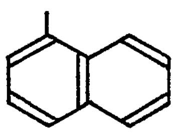 | 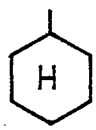 | $CH_2-S-TZ$ |
| $(CH_3)_3COCONH$ | 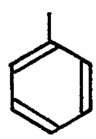 | 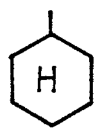 | $CH_2-S-TZ$ |
| $(CH_3)_3COCONH$ | 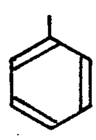 | 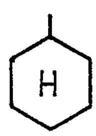 | $CH_2-S-TZ$<br>$CH_2CONH_2$ |

| | | | |
|---|---|---|---|
| $(CH_3)_3COCONH$ | 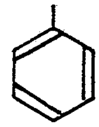 | 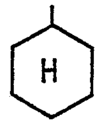 | 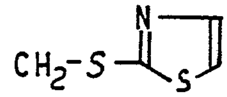 |
| $(CH_3)_3COCONH$ | 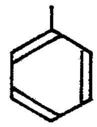 | 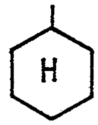 | 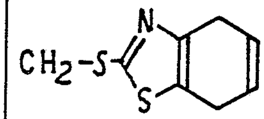 |
| $(CH_3)_3COCONH$ | 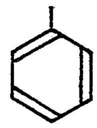 | 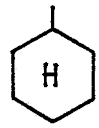 | 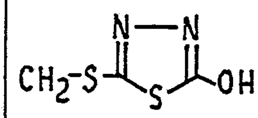 |
| 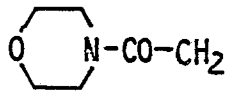 | 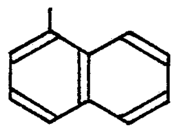 | 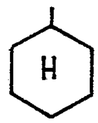 | 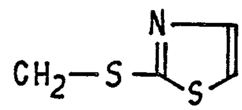 |
| 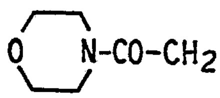 | 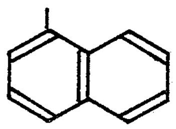 | 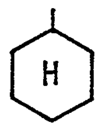 | 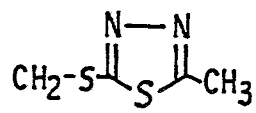 |
| 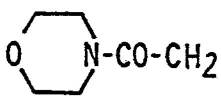 | 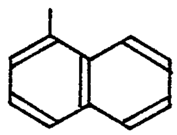 | 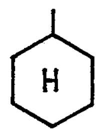 | 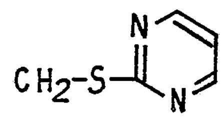 |
| 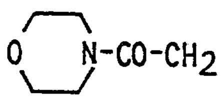 | 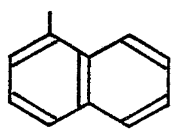 | 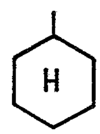 | 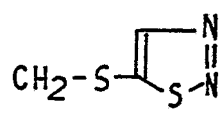 |
| 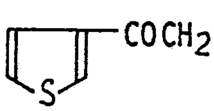 | 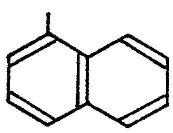 | 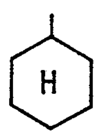 | $CH_2S—TZ$ |

| | | | |
|---|---|---|---|
| $(CH_3)_3COCONH$ | 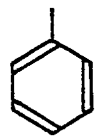 | 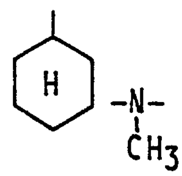 | 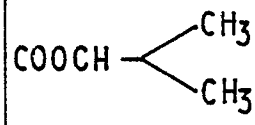 |
| $(CH_3)_3COCONH$ | 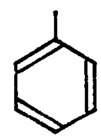 | 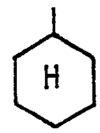 | 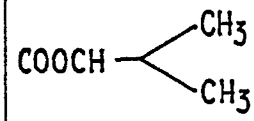 |
| 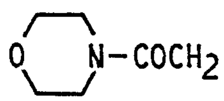 | 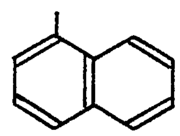 | 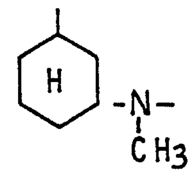 | 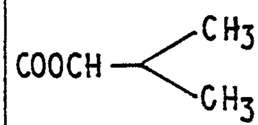 |

Compounds of formula I and their salts have been found to exhibit the characteristics (1) to (4) mentioned in the introduction to this specification.

**Claims**

1. A compound of formula (I) or a salt thereof

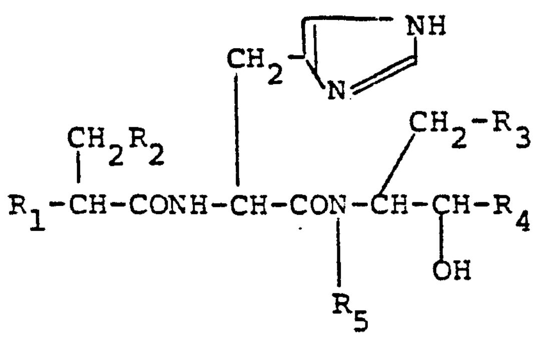

(I)

wherein $R_1$ is a ($C_1$-$C_6$ alkoxy)carbonyl or ($C_1$-$C_6$ alkoxy)carbonylamino group, a group of formula $-(CH_2)_n$-A-$R^a$ [wherein n is an integer of 1 or 2: A is a single bond between $-(CH_2)_n$ and $R^a$, an alkylene group which may be substituted by hydroxy group(s), or a carbonyl group; and $R^a$ is a cyano, $C_1$-$C_6$ alkyl, $C_1$-$C_6$ alkynyl, cycloalkyl, cylcoalkyidene, or aryl group, an aryloxy group which may be substituted by halogen atom(s), or a heterocyclic group which may be substituted by amino group(s)], or a group of formula -CONH-B-$R^b$-[wherein B is a single bond, or alkylene; and $R^b$ is a hydroxyl, aryl, or heterocyclic group]; $R_2$ is a phenyl or naphthyl group; $R_3$ is a $C_1$-$C_6$ alkyl group or a cyclohexyl or phenyl group: $R_4$ is a nitromethyl group, a group of formula -COOR$^c$ (wherein $R^c$ is a $C_1$-$C_6$ alkyl group), or a group of formula

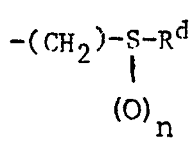

(wherein n is zero or an integer of 1 or 2 and $R^d$ is a heterocyclic group which may be substituted by at least one group selected from $C_1$-$C_6$ alkyl, carbamoyl-($C_1$-$C_6$ alkyl) and hydroxy-($C_1$-$C_6$ alkyl) groups]: and $R_5$ is a hydrogen atom or a $C_1$-$C_6$ alkyl group.

2. A compound according to claim 1 which is (2RS, 3S)-3-[N-[2-(1-naphthylmethyl)-3-(morpholinocarbonyl)-propionyl]-L-histidyl]amino-2-hydroxy-4-cyclohexylbutryic acid isopropyl ester; or

(RS,3S)-3-[n-[1,4-dioxo-4-morpholino-2-(1-naphthylmethyl)-butyl-L-histidylamino)]-4-cyclohexyl-1-(1-methyl-5-tetrazolylthio]-2-butanol; or

(2RS,3S)-3-(N-t-butoxycarbonyl-L-phenylalanyl-L-histidylamino-4-cyclohexyl-1-(1-methyl-5-tetrazolylthio)-2-butanol; or a salt of any of these

3. A pharmaceutical composition containing any compound(s) according to claim 1 or 2.

4. The use of a compound according to claim 1 or 2 for the preparation of a medicament having renin-inhibitor action.

5. A process for producing a compound according to claim 1 or 2 substantially as described hereinbefore in any of Processes 1 to 3, e.g. substantially as described in any Example.

6. A compound according to claim 1 which as a defined herein in the Table following the Examples.

7. An intermediate compound of any of formulae II to VII herein.

8. An intermediate compound which is the product of any of Reference Examples 1 to 61.